(19) **Europäisches Patentamt / European Patent Office / Office européen des brevets**

(11) **EP 4 592 269 A1**

(12) **EUROPEAN PATENT APPLICATION**
published in accordance with Art. 153(4) EPC

(43) Date of publication:
30.07.2025 Bulletin 2025/31

(21) Application number: 23868149.8

(22) Date of filing: 15.09.2023

(51) International Patent Classification (IPC):
*C04B 35/488* (2006.01)    *A61C 13/083* (2006.01)
*C01G 25/02* (2006.01)

(52) Cooperative Patent Classification (CPC):
A61C 13/083; A61K 6/802; A61K 6/813;
A61K 6/816; A61K 6/818; A61K 6/822;
A61K 6/824; C01G 25/02; C04B 35/488

(86) International application number:
PCT/JP2023/033785

(87) International publication number:
WO 2024/063028 (28.03.2024 Gazette 2024/13)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 20.09.2022  JP 2022149612
10.03.2023  JP 2023038003

(71) Applicant: Tosoh Corporation
Yamaguchi 746-8501 (JP)

(72) Inventors:
• USHIO Yuki
Shunan-shi Yamaguchi 746-8501 (JP)
• TSUKIMORI Takashi
Shunan-shi Yamaguchi 746-8501 (JP)
• NAGAYAMA Hitoshi
Shunan-shi Yamaguchi 746-8501 (JP)

(74) Representative: Müller-Boré & Partner
Patentanwälte PartG mbB
Friedenheimer Brücke 21
80639 München (DE)

(54) **ZIRCONIA COMPOSITION AND METHOD FOR PRODUCING SAME**

(57)    Provided is at least one selected from a calcined body that is easier to work than existing calcined bodies and that has a smaller difference in hardness between the production lots, a method for producing the same, a sintered body obtained therefrom and a zirconia compositional substance used as a raw material of this calcined body. A zirconia compositional substance comprises at least one first transition metal element selected from the group consisting of manganese (Mn), iron (Fe), cobalt (Co), nickel (Ni), copper (Cu), molybdenum (Mo), technetium (Tc), ruthenium (Ru), rhodium (Rh), palladium (Pd) and silver (Ag); a coloring element that is either or both of a lanthanoid rare earth element and a second transition metal element different from the first transition metal element; and stabilizing element-containing zirconia, wherein a content of the first transition metal element is 100 mass ppm or more, a content of the second transition metal element is less than 100 mass ppm, and the zirconia compositional substance satisfies either or both of the following conditions: when a characteristic X-ray of a zirconium element therein and a characteristic X-ray of the first transition metal element are measured, a percentage of measurement points at which a ratio of an intensity of the characteristic X-ray of the first transition metal element to an intensity of the characteristic X-ray of the zirconium element is 0.05 or more is 3% or less of all measurement points; and when the characteristic X-ray of the zirconium element therein and the characteristic X-ray of the first transition metal element are measured, a distribution width of the ratio of the intensity of the characteristic X-ray of the first transition metal element to the intensity of the characteristic X-ray of the zirconium element is 0.3 or less.

EP 4 592 269 A1

## Description

### TECHNICAL FIELD

The present disclosure relates to a compositional substance containing zirconia as a main component and a method for producing the same.

### BACKGROUND ART

Zirconia ($ZrO_2$, zirconium dioxide) sintered bodies containing color components have found a wide variety of usage including portable electronic appliance members, decorative members, and dental prostheses. Zirconia sintered bodies have high strength and are difficult to work. Thus, in order to obtain a complex-shape sintered body such as a dental prosthesis, a calcined body (semi-sintered body or pre-sintered body), which is zirconia that is obtained by heat-treating a zirconia green body (compact) at a temperature lower than the sintering temperature and that has a strength suitable for working, is used. The calcined body is machined into a desired shape by using CAD/CAM and then sintered into a sintered body.

An example of a method for obtaining a sintered body having a color of natural teeth is a method involving sintering a zirconia calcined body dipped in a color solution (that is, a dipping method) (for example, Patent document 1). A calcined body colored by the dipping method has color unevenness caused by the difference in the degree of permeation of the color solution between the surface and the inside of the calcined body. In addition, it has been difficult by the dipping method to obtain subtle color tones that match the color tones of the natural teeth of individual patients.

Meanwhile, there is known a method that involves forming and calcining a powder compositional substance obtained by mixing a color component and zirconia in a powder state and then sintering the calcined body that already contains the color component as calcined (also known as a powder mixing method) (Patent document 2). The calcined body obtained by the powder mixing method has significantly less color unevenness compared to the calcined body obtained by the dipping method, and it is easy to finely adjust the color tone of the sintered body to be obtained.

### PRIOR ART DOCUMENTS

### PATENT DOCUMENTS

Patent Document 1: European Patent Application Publication No. 3892254
Patent Document 2: United States Patent No. 9428422

### DISCLOSURE OF INVENTION

### TECHNICAL PROBLEM

The color tone of a sintered body obtained from a calcined body is determined by adjusting the composition, in particular, the type and content of the color component. Meanwhile, the color component affects the thermal shrinkage behavior of the zirconia compositional substance even if the amount thereof is very small. Thus, zirconia compositional substances having different compositions have thermal shrinkage behaviors different from each other. As a result, calcined bodies that give sintered bodies having different color tones have had hardness different from each other. Calcined bodies having different hardness values apply different loads on the working machine, and thus a well-trained working technique is necessary. Moreover, the higher the hardness, the more difficult it is to work the calcined body.

In addition to the difference in workability derived from the difference in composition, there has been cases in which the difference in hardness is found between production lots of calcined bodies obtained by calcining green bodies containing color components despite having the same composition. The difference in hardness derived from the difference in composition can be reduced to some degree by adopting different calcining conditions according to the composition (color tone) of the calcined body. However, it is not preferable to change the calcining conditions according to the composition of the calcined body since this complicates the process of producing the calcined body. Furthermore, for the calcined bodies of the same composition, the difference in hardness between the production lots cannot be reduced even when multiple calcining conditions are adopted.

In view of these issues, an object of the present disclosure is to provide at least one selected from a calcined body that is easier to work than existing calcined bodies and that has a smaller difference in hardness between the production lots, a method for producing the same, a sintered body obtained therefrom and a zirconia compositional substance used as a raw material of this calcined body.

SOLUTION TO PROBLEM

The inventors of the present disclosure have studied in detail the thermal shrinkage behavior and the composition of the zirconia compositional substance used as a precursor of a calcined body. As a result, it has been found that, of the color components, transition metal elements have a large influence on the thermal shrinkage behavior of the zirconia compositional substance and that the thermal shrinkage behavior changes by controlling the state of the transition metal elements contained in a particular amount or more without changing the essential composition. As a result, a calcined body from which a sintered body having a color tone similar to a sintered body obtained from an existing calcined body can be obtained and with which the difference in hardness of the calcined bodies of different production lots is reduced, and a zirconia compositional substance that serves as a precursor of such a calcined body has been found.

That is, the present invention is as disclosed by the claims, and the gist of the present disclosure is as follows.

[1] A zirconia compositional substance comprising:

at least one first transition metal element selected from the group consisting of manganese (Mn), iron (Fe), cobalt (Co), nickel (Ni), copper (Cu), molybdenum (Mo), technetium (Tc), ruthenium (Ru), rhodium (Rh), palladium (Pd) and silver (Ag);
a coloring element that is either or both of a lanthanoid rare earth element and a second transition metal element different from the first transition metal element; and
stabilizing element-containing zirconia,

wherein

a content of the first transition metal element is 100 mass ppm or more,
a content of the second transition metal element is less than 100 mass ppm, and
the zirconia compositional substance satisfies either or both of the following conditions:

when a characteristic X-ray of a zirconium element therein and a characteristic X-ray of the first transition metal element are measured, a percentage of measurement points at which a ratio of an intensity of the characteristic X-ray of the first transition metal element to an intensity of the characteristic X-ray of the zirconium element is 0.05 or more is 3% or less of all measurement points; and
when the characteristic X-ray of the zirconium element therein and the characteristic X-ray of the first transition metal element are measured, a distribution width of the ratio of the intensity of the characteristic X-ray of the first transition metal element to the intensity of the characteristic X-ray of the zirconium element is 0.3 or less.

[2] The zirconia compositional substance described in [1] above, wherein, when the characteristic X-ray of the zirconium element therein and the characteristic X-ray of the first transition metal element are measured, the distribution width of the ratio of the intensity of the characteristic X-ray of the first transition metal element to the intensity of the characteristic X-ray of the zirconium element is 0.3 or less.

[3] The zirconia compositional substance described in [1] or [2] above, wherein, when the characteristic X-ray of the zirconium element therein and the characteristic X-ray of the first transition metal element are measured, the percentage of measurement points at which the ratio of the intensity of the characteristic X-ray of the first transition metal element to the intensity of the characteristic X-ray of the zirconium element is 0.05 or more is 3% or less of all measurement points.

[4] The zirconia compositional substance described in any one [1] to [3] above, wherein the second transition metal element is at least one transition metal element selected from the group consisting of manganese, iron, cobalt, nickel, copper, molybdenum, technetium, ruthenium, rhodium, palladium and silver.

[5] The zirconia compositional substance described in any one of [1] to [4] above, wherein the lanthanoid rare earth element is at least one selected from the group consisting of praseodymium (Pr), neodymium (Nd), samarium (Sm), europium (Eu), gadolinium (Gd), terbium (Tb), erbium (Er) and ytterbium (Yb).

[6] The zirconia compositional substance described in any one of [1] to [5] above, wherein the lanthanoid rare earth element is a lanthanoid rare earth element dissolved in zirconia.

[7] The zirconia compositional substance described in any one of [1] to [6] above, wherein a content of the lanthanoid rare earth element is 1.0 mass% or less.

[8] The zirconia compositional substance described in any one of [1] to [7] above, comprising at least one third transition metal element selected from the group consisting of titanium (Ti), vanadium (V) and niobium (Nb).

[9] The zirconia compositional substance described in any one of [1] to [8] above, comprising at least one selected from the group consisting of alumina ($Al_2O_3$), silica ($SiO_2$) and germania ($Ge_2O_3$).

[10] The zirconia compositional substance described in any one of [1] to [9] above, wherein a BET specific surface area is 8 $m^2$/g or more and 15 $m^2$/g or less.

[11] The zirconia compositional substance described in any one of [1] to [10] above, wherein the zirconia compositional substance is a powder.

[12] The zirconia compositional substance described in [11] above, wherein, when 3.0 $\pm$ 0.1 g of the zirconia compositional substance is charged in a mold having a diameter of 25 mm, uniaxially press-formed at a pressure of 49 MPa and then subjected to a CIP process at a pressure of 196 MPa, a measured density is 3.2 $g/cm^3$ or more.

[13] The zirconia compositional substance described in any one of [1] to [10] above, wherein the zirconia compositional substance is a green body.

[14] The zirconia compositional substance described in [13] above, wherein the zirconia compositional substance has Vickers hardness of 11.0 or more.

[15] A method for producing a calcined body, the method comprising a step of calcining the zirconia compositional substance described in any one of [1] to [14] above.

[16] A method for producing a sintered body, the method comprising a step of sintering a calcined body obtained by the production method described in [15] above.

ADVANTAGEOUS EFFECTS OF INVENTION

The present disclosure can provide at least one selected from a calcined body that is easier to work than existing calcined bodies and that has a smaller difference in hardness between the production lots, a method for producing the same, a sintered body obtained therefrom and a zirconia compositional substance used as a raw material of such a calcined body.

DESCRIPTION OF EMBODIMENTS

A zirconia compositional substance according to the present disclosure will now be described through some exemplary embodiments. The terms used in the embodiments are as follows.

A "compositional substance" is a substance having a particular composition, and is, for example, at least one selected from the group consisting of a powder, a green body, a calcined body and a sintered body. A "zirconia compositional substance" is a compositional substance containing zirconia as a main component, in particular, a compositional substance essentially consisting of zirconia. This means that the components other than zirconia may be contained. The zirconia compositional substance according to this embodiment is preferably a compositional substance that can serve as a precursor of a calcined body.

A "powder" is a set of powder particles (particles that are either or both of primary particles and secondary particles) and is a compositional substance having flowability. A "zirconia powder" is a powder containing zirconia as a main component, in particular, a powder essentially consisting of zirconia. Furthermore, a "powder compositional substance" is a compositional substance constituted by powders having different features, in particular, a compositional substance containing powders having different compositions.

A "granular powder" is a set of aggregates (granular particles) of powder particles and is a compositional substance having flowability, in particular, a compositional substance in a state where the powder particles have slowly aggregated. A "zirconia granular powder" is a granular powder containing zirconia as a main component, in particular, a granular powder essentially consisting of zirconia.

A "green body" is a compositional substance that has a particular shape constituted by powder particles aggregated by a physical force, in particular, a compositional substance in a state where heat treatment is not yet performed after the shape is given (for example, after forming). A "zirconia green body" is a green body containing zirconia as a main component, in particular, a green body essentially consisting of zirconia. In addition, the green body is interchangeable with a "compact".

A "calcined body" is a compositional substance that has a particular shape constituted by fused particles, in particular, a compositional substance heat-treated at a temperature lower than a sintering temperature. A "zirconia calcined body" is a calcined body containing zirconia as a main component, in particular, a calcined body essentially consisting of zirconia.

A "sintered body" is a compositional substance that has a particular shape constituted by crystal grains, in particular, a compositional substance heat-treated at a temperature higher than or equal to the sintering temperature. A "zirconia sintered body" is a sintered body containing zirconia as a main component, in particular, a sintered body essentially consisting of zirconia.

A "main component" is a component that constitutes a main phase (matrix, base material or base phase) in the composition of the compositional substance, and the mass ratio of the main component relative to the compositional substance is preferably 75 mass% or more, 85 mass% or more, 90 mass% or more, 95 mass% or more, 98 mass% or more or 99 mass% or more and 100 mass% or less or less than 100 mass%, and preferably 75 mass% or more and 100 mass% or less or 95 mass% or more and less than 100 mass%.

A "stabilizing element" is an element that stabilizes zirconia crystal phases by dissolving into zirconia.

A "BET specific surface area" is a specific surface area [$m^2/g$] measured in accordance with JIS R 1626 by a BET multipoint method (5 points) using nitrogen as adsorption gas, in particular, a BET specific surface area measured under the following conditions.

Adsorption medium: $N_2$
Adsorption temperature: -196°C
Pretreatment conditions: air atmosphere, degassing treatment at 250°C for 1 hour or longer

The BET specific surface area can be measured by using a typical specific surface area meter (for example, TriStar II 3020 produced by Shimadzu Corporation).

Note that, in this embodiment, the "air atmosphere" is mainly composed of nitrogen and oxygen, is, for example, a nitrogen atmosphere having an oxygen concentration of 18 to 23 vol%, and may contain moisture.

An "average particle size" is D50 in a volume-based particle size distribution of a powder measured by a wet method, and can be measured with a generally available laser diffraction/scattering particle size distribution meter (for example, MT3300EXII produced by MicrotracBEL Corp.). A measurement sample may be a slurry prepared by removing slow aggregates from a powder by a dispersion process such as an ultrasonic process and dispersing the resulting powder in pure water. The volume-based particle size distribution measurement by a wet method is preferably performed by adjusting the pH of the slurry to 3.0 to 6.0.

An "average granule size" is D50 in a volume-based particle size distribution of a granular powder measured by a dry method, and can be measured with a generally available laser diffraction/scattering particle size distribution meter (for example, MT3100II produced by MicrotracBEL Corp.). The measurement sample may be a granular powder in a slow aggregation state without being subjected to a dispersing treatment such as an ultrasonic treatment.

A "powder X-ray diffraction pattern" is an X-ray diffraction (hereinafter may also be referred to as "XRD") pattern obtained by performing smoothing and background subtraction on an XRD pattern, which is obtained from the compositional substance by powder XRD measurement under the following conditions, by using an analyzing program that comes with an X-ray diffractometer (for example, Integrated Analytical Software for Powder X-ray Diffraction, PDXL Ver. 2.2 produced by RIGAKU Corporation).

Radiation source: CuK$\alpha$ radiation ($\lambda$ = 0.15418 nm)
Measurement mode: continuous scan
Scan speed: 2°/minute
Measurement range:

$2\theta$ = 26° to 33°
$2\theta$ = 72° to 76°

Accelerating voltage/current: 40 mA/40 kV
Divergence height slit: 10 mm
Divergence/incident slit: 1°
Receiving slit: open
Detector: semiconductor detector (D/teX Ultra)
Filter: Ni filter
Goniometer radius: 185 mm

XRD measurement can be carried out by using a common X-ray diffractometer (for example, Ultima IV produced by RIGAKU Corporation). The calcined body may have surfaces polished with a #400 grit sandpaper in accordance with JIS R 6001-2 and then lap-polished by using a 3 $\mu$m grit diamond abrasive to prepare a measurement sample, and the XRD measurement may be performed on the lap-polished surface. A measurement sample may be prepared by polishing surfaces of a sintered body to a surface roughness of Ra $\leq$ 0.02 $\mu$m, and the XRD measurement may be performed on the polished surface.

An "XRD peak" is a peak that has a peak top at 2θ detected in an XRD pattern obtained by the aforementioned XRD measurement. In this embodiment, "having no XRD peak" means that the XRD peak is not detected in the aforementioned XRD measurement.

The XRD peaks corresponding to the crystal planes of zirconia are those XRD peaks that have peak tops at 2θ below:

XRD peak corresponding to monoclinic (111) plane: 2θ = 31 ± 0.5°
XRD peak corresponding to monoclinic (11-1) plane: 2θ = 28 ± 0.5°
XRD peak corresponding to tetragonal (111) plane: 2θ = 30 ± 0.5°
XRD peak corresponding to cubic (111) plane: 2θ = 30 ± 0.5°

The XRD peak corresponding to the tetragonal (111) plane and the XRD peak corresponding to the cubic (111) plane are measured as one overlapping peak.

A "T + C phase ratio" is a ratio of the area intensity of the XRD peak of the tetragonal and cubic zirconia relative to the total area intensity of the XRD peaks of the tetragonal, cubic, and monoclinic zirconia in the XRD pattern obtained in the aforementioned XRD measurement, and is determined from the following equation.

$$f_{T+C} = [I_t(111) + I_c(111)] / [I_m(111) + I_m(11\text{-}1) + I_t(111) + I_c(111)]$$

In the equation above, $f_{T+C}$ represents the tetragonal and cubic phase ratio, $I_t(111)$ represents the area intensity of the tetragonal (111) plane, $I_c(111)$ represents the area intensity of the cubic (111) plane, $I_m(111)$ represents the area intensity of the monoclinic (111) plane, $I_m(11\text{-}1)$ represents the area intensity of the monoclinic (11-1) plane, and $I_t(111) + I_c(111)$ corresponds to the area intensity of the XRD peak that has a peak top at 2θ = 30 ± 0.5°.

The area intensity of each XRD peak is a value obtained by analyzing the XRD pattern by using an analyzing program that comes with an X-ray diffractometer (for example, Integrated Analytical Software for Powder X-ray Diffraction, PDXL Ver. 2.2 produced by RIGAKU Corporation).

A "measured density" is a value [g/cm$^3$] of the mass [g] relative to the sample volume [cm$^3$]. The mass may be a value obtained by weighing the sample. The volume may be a volume determined by measuring the dimensions for the green body and the calcined body and may be a volume determined by Archimedes' principle in accordance with JIS R 1634 for the sintered body. Determination by Archimedes' principle may be conducted by using ion exchange water as a solvent and a boiling method as a pretreatment. The measured densities of the green body, the calcined body and the sintered body are respectively referred to as the "green body density", the "calcined body density" and the "sintered body density".

A "Vickers hardness" is a value measured by using a common Vickers hardness tester (for example, Q30A produced by Qness GmbH) equipped with a square-based pyramid diamond indenter. In the measurement, the indenter is statically pressed into a measurement sample surface, and the diagonal length of the indentation mark formed in the measurement sample surface is measured. The obtained diagonal length is used to determine the Vickers hardness from the equation below.

$$Hv = F/\{d^2/2\sin(\alpha/2)\}$$

In the equation above, Hv represents the Vickers hardness (HV), F represents a measurement load (1 kgf), d represents the diagonal length (mm) of the indentation mark, and α represents the face angle (136°) of the indenter.

The examples of the measurement conditions for the Vickers hardness are as follows.

Measurement sample: a disk shape with a thickness of 3.0 ± 0.5 mm
Measurement load: 1 kgf

Prior to the measurement, the measurement sample may be subjected to a pretreatment by polishing the measurement surface with a #800 water-resistant abrasive paper to remove irregularities exceeding 0.1 mm. Measurement may be conducted on ten points in the measurement sample and the average thereof may be assumed to be the Vickers hardness.

A "total transmittance" is the ratio [%] of transmitted light (total of regular transmitted light and diffuse transmitted light) relative to incident light measured in accordance with JIS K 7361-1. A disk-shaped sintered body having a sample thickness of 1.0 ± 0.1 mm and a surface roughness Ra ≤ 0.02 μm in both surfaces may be used as the measurement sample, and a haze meter (for example, haze meter NDH4000 produced by NIPPON DENSHOKU INDUSTRIES Co.,

Ltd.) equipped with a D65 light source as the light source may be used as the measurement instrument.

The "color tone (L\*, a\*, b\*)" is a value measured by an SCI system with a spectrophotometric colorimeter (for example, CM-700d produced by KONICA MINOLTA JAPAN, INC.) equipped with an Illumination/Receiving optical system in conformity with the geometrical condition c of JIS Z 8722. Specific conditions for measuring the color tone by a method involving placing a white calibration plate as the background of the measurement sample (also known as white background measurement) are the following conditions. The color tone of a sintered body may be measured by cutting out a desired part of the sintered body in a horizontal direction, processing the resulting cut part to a sample thickness of $1.0 \pm 0.1$ mm, and measuring the color tone of this measurement sample.

Light source: D65 light source
Viewing angle: 2°
Measurement system: SCI

The lightness L\* is an index of the brightness and has a value of 0 or more and 100 or less. The hue a\* and b\* is an index of the shade of color and has a value of - 100 or more and 100 or less. The saturation C\* is an index of vividness.

The "three-point flexural strength" is a value measured in conformity with JIS R 1601. The measurement sample used may have a columnar shape having a width of 4 mm, a thickness of 3 mm and a length of 45 mm, and a load may be applied in a horizontal direction of the measurement sample with a support span of 30 mm.

"Pressureless sintering" is a method for sintering a material to be sintered (such as a green body or calcined body) by heating the material at a temperature (hereinafter may also be referred to as a "sintering temperature") at which sintering of zirconia proceeds without applying external force to the material.

"Pressureless firing" is a method for heating a material to be treated without adding external force during heat treatment at a temperature lower than the sintering temperature.

[Zirconia compositional substance]

This embodiment involves a zirconia composition comprising at least one first transition metal element selected from the group consisting of manganese (Mn), iron (Fe), cobalt (Co), nickel (Ni), copper (Cu), molybdenum (Mo), technetium (Tc), ruthenium (Ru), rhodium (Rh), palladium (Pd) and silver (Ag),

a coloring element that is either or both of a lanthanoid rare earth element and a second transition metal element different from the first transition metal element and
stabilizing element-containing zirconia,
wherein the content of the first transition metal element is 100 mass ppm or more,
the content of the second transition metal element is less than 100 mass ppm, and
the zirconia composition satisfies at least one of the following conditions:

when a characteristic X-ray of a zirconium element therein and a characteristic X-ray of the first transition metal element are measured, a percentage of measurement points at which a ratio of an intensity of the characteristic X-ray of the first transition metal element to an intensity of the characteristic X-ray of the zirconium element is 0.05 or more is 3% or less of all measurement points; and
when the characteristic X-ray of the zirconium element therein and the characteristic X-ray of the first transition metal element are measured, a distribution width of the ratio of the intensity of the characteristic X-ray of the first transition metal element to the intensity of the characteristic X-ray of the zirconium element is 0.3 or less. The first transition metal element (hereinafter may also be referred to as the "M1 element") has a function to color zirconia, forms the base of the color tone, and is a transition metal element that is contained in a large amount in the zirconia compositional substance of this embodiment and preferably a transition metal element that is contained in the largest amount in the zirconia compositional substance of this embodiment. The M1 element has a strong influence on the thermal shrinkage behavior of the zirconia compositional substance and is prone to segregate after sintering. However, since the influence of the M1 element on the thermal shrinkage behavior is drastically reduced as a result of satisfying the features of the zirconia compositional substance of this embodiment, the zirconia compositional substance of this embodiment can serve as a precursor of calcined bodies in which the difference in mechanical strength between the production lots is reduced.

The zirconia compositional substance of this embodiment can serve as a precursor of a calcined body and is preferably either or both of a zirconia powder and a zirconia green body. Alternatively, the zirconia compositional substance of this embodiment may be a zirconia green body or a zirconia powder.

The zirconia compositional substance of this embodiment contains stabilizing element-containing zirconia. The stabilizing element-containing zirconia is preferably zirconia having a stabilizing element dissolved therein (stabilizing element-dissolved zirconia). Thus, the zirconia compositional substance of this embodiment may be considered a stabilizing element-containing zirconia compositional substance or a stabilizing element-dissolved zirconia compositional substance.

The stabilizing element may be any element that dissolves in zirconia without coloring zirconia, for example, at least one selected from the group consisting of yttrium (Y), calcium (Ca) and magnesium (Mg), and is preferably yttrium.

The content of the stabilizing element in the zirconia compositional substance of this embodiment (hereinafter this content may also be referred to as the "stabilizing element amount", and when the stabilizing element is yttrium or the like, the content may be referred to as the "yttrium amount") may be any amount at which the crystal phases of zirconia are stably composed of crystal phases including the tetragonal phase as the main phase or crystal phases composed of the tetragonal phase and the cubic phase, for example, 2.0 mol% or more and 8.0 mol% or less. When the stabilizing element is yttrium, the stabilizing element amount (yttrium amount) may be, for example, 2.6 mol% or more, 3.1 mol% or more, 3.6 mol% or more or 3.9 mol% or more and 6.0 mol% or less, 5.6 mol% or less, 5.4 mol% or less, 4.3 mol% or less or 4.1 mol% or less and is preferably 2.6 mol% or more and 6.0 mol% or less, 3.6 mol% or more and 5.4 mol% or less or 3.9 mol% or more and 4.1 mol% or less.

In this embodiment, the stabilizing element amount is the molar ratio [mol%] of the stabilizing element in terms of oxide relative to the total of zirconia and the stabilizing element and the lanthanoid rare earth element in terms of oxides. The oxide of the stabilizing elements used in the conversion is $Y_2O_3$ for yttrium, CaO for calcium and MgO for magnesium.

The zirconia compositional substance of this embodiment preferably does not contain undissolved stabilizing elements. Absence of the undissolved stabilizing elements can be confirmed through the absence of the XRD peaks derived from the compounds of the stabilizing elements in the XRD pattern. However, so long as the effects of the zirconia compositional substance of this embodiment are exhibited, inclusion of the undissolved stabilizing elements, that is, inclusion of the undissolved stabilizing elements in the range where the XRD peaks derived from the compounds of the stabilizing elements in the XRD pattern are not detected, is allowable.

The zirconia compositional substance of this embodiment contains at least one M1 element selected from the group consisting of manganese (Mn), iron (Fe), cobalt (Co), nickel (Ni), copper (Cu), molybdenum (Mo), technetium (Tc), ruthenium (Ru), rhodium (Rh), palladium (Pd) and silver (Ag). The M1 element has a function of coloring zirconia and is a transition metal element that is prone to segregate after sintering. The M1 element is an element that functions as a color component (main color component) that affects the base of the color tone of the sintered body obtained from the zirconia compositional substance of this embodiment. In order to obtain a sintered body of a color tone suitable for the dental prostheses, the zirconia compositional substance of this embodiment preferably contains one transition metal element that is contained in an amount of 100 mass ppm or more, in other words, one M1 element. Meanwhile, when two or more transition metal elements are contained in an amount of 100 mass ppm or more, for the sake of convenience, the transition metal element contained in the largest amount among the transition metal elements is referred to as the M1 element, and other M1 elements may be distinguished and referred to as a transition metal element 1' (M1' element), a transition metal element 1" (M1" element) etc.

The content of the M1 element (M1 element amount) in the zirconia compositional substance of this embodiment is 100 mass ppm or more, and the color tone derived from the M1 element intensifies as the content thereof increases. The M1 element amount is 150 mass ppm or more, 300 mass ppm or more or 500 mass ppm or more and 0.2 mass% or less (2000 mass ppm or less), 0.15 mass% or less, 0.12 mass% or less or 0.1 mass% or less (1000 mass ppm or less), for example. The specific M1 element amount may be adjusted as appropriate for the target color tone. Examples of the M1 element amount for achieving the color tone suitable for dental prostheses include 100 mass ppm or more and 1500 mass ppm or less, 100 mass ppm or more and 1000 mass ppm or less and 100 mass ppm or more and 500 mass ppm or less.

The M1 element amount in this embodiment is the mass ratio [mass ppm] of the M1 element in terms of oxide relative to the total mass (hereinafter may also be referred to as the "metal element mass") of zirconia and metal elements in terms of oxides.

The type of the M1 element may be selected as appropriate for the target color tone, and examples thereof include at least one selected from the group consisting of manganese, iron, cobalt, nickel, copper, molybdenum and silver, at least one selected from the group consisting of manganese, iron, cobalt, nickel and copper, at least one selected from the group consisting of manganese, iron and nickel, either or both of manganese and iron, either or both of iron and nickel and iron. The M1 element may be manganese, iron, cobalt, nickel, copper, molybdenum or silver, may be manganese, iron, cobalt or nickel, or may be nickel or iron. For example, when a sintered body having a gray-based color tone is to be obtained, the M1 element is manganese; when a sintered body having a yellow-based color tone is to be obtained, the M1 element is iron; when a sintered body having a blue-based color tone is to be obtained, the M1 element is cobalt; and when a sintered body having a green-based color tone is to be obtained, the M1 element is nickel.

The zirconia compositional substance of this embodiment may contain the M1 element in any form. The M1 element is, for example, contains as at least one selected from the group consisting of an oxide, a hydroxide, an oxyhydroxide, a halide, a sulfate, a nitrate and an acetate, at least one selected from the group consisting of an oxide, a hydroxide, an oxyhydroxide and a chloride, either or both of an oxide and an oxyhydroxide or an oxide.

The zirconia compositional substance of this embodiment satisfies either or both of the following conditions: when the characteristic X-ray of the zirconium element and the characteristic X-ray of the M1 element are measured, the percentage of measurement points at which the ratio (hereinafter may also be referred to as the "X-ray intensity ratio") of the intensity of the characteristic X-ray of the M1 element to the intensity of the characteristic X-ray of the zirconium element is 0.05 or more is 3% or less of all measurement points (in other words, the number of the measurement points at which the X-ray intensity ratio is 0.05 or more is 3% or less of the number of all measurement points) and that, when the characteristic X-ray of the zirconium element and the characteristic X-ray of the M1 element are measured, the distribution width of the ratio of the intensity of the characteristic X-ray of the M1 element to the intensity of the characteristic X-ray of zirconium element is 0.3 or less. Presumably because such conditions are satisfied, localization of the M1 element is significantly reduced. Presumably as a result, local rapid thermal shrinkage caused by inhomogeneous presence of the M1 element is reduced, the hardness of the calcined body obtained is decreased, and variation in thermal shrinkage behavior caused by the difference between the production lots is reduced. Thus, the difference in hardness between the production lots of the calcined bodies obtained from the zirconia compositional substance of this embodiment can be reduced.

The percentage of the measurement points at which the X-ray intensity ratio is 0.05 or more (hereinafter this percentage is also referred to as the "high-intensity percentage") relative to all measurement points is preferably 3% or less, 2% or less, 0.5% or less, 0.1% or less or 0.05% or less. The high-intensity percentage may be 0% or more, more than 0% or 0.01% or more and is preferably 0% or more and 3% or less, more than 0% and 3% or less or 0.01% or more and 0.5% or less.

For the zirconia compositional substance that satisfies the intensity ratio range described below, the high-intensity percentage may be any desired value, for example, 0% or more and less than 100%, 50% or more and 99.5% or less or 80% or more and 99.5% or less.

The zirconia compositional substance of this embodiment is preferably free of coarse aggregation of the M1 element, and the percentage of the measurement points at which the X-ray intensity ratio is 0.1 or more (hereinafter this percentage is also referred to as the "coarse intensity percentage") relative to all measurement points is preferably 10% or less, 8% or less, 5% or less, 0.5% or less, 0.3% or less, 0.1% or less or 0.01% or less. The coarse intensity percentage may be 0% or more or more than 0%, and is preferably 0% or more and 10% or less, more than 0% and 10% or less or more than 0% and 5% or less. For example, the coarse intensity percentage when the M1 element is iron is 0% or more and 0.5% or less or 0% or more and 0.01% or less.

The distribution width of the X-ray intensity ratio of the zirconia compositional substance of this embodiment (that is, the absolute value of the difference between the maximum intensity ratio (described below) and the minimum intensity ratio (described below), hereinafter may also be referred to as the "intensity ratio range") is preferably 0.3 or less, 0.2 or less, 0.15 or less, 0.12 or less, 0.1 or less or 0.08 or less. In this manner, the obtained calcined bodies are likely to have more homogeneous hardness.

A narrow intensity ratio range indicates that the values of the X-ray intensity ratios at all measurement points are similar to one another, that is, that the M1 element is present in the compositional substance in a more homogeneous state. Presumably as a result, local rapid thermal shrinkage caused by inhomogeneous presence of the M1 element is reduced, the hardness of the calcined body obtained is decreased, and variation in thermal shrinkage behavior caused by the difference between the production lots is reduced. Thus, the zirconia compositional substance of this embodiment preferably has an intensity ratio range of the aforementioned values in addition to or instead of having a high-intensity percentage of 3% or less. When the zirconia compositional substance of this embodiment exhibits such an intensity ratio range, the influence of the M1 element on the thermal shrinkage behavior of the zirconia compositional substance becomes more homogeneous. The intensity ratio range may be more than 0, 0.01 or more, 0.04 or more or 0.05 or more. The intensity ratio range is, for example, more than 0 and 0.3 or less, more than 0 and 0.2 or less, 0.01 or more and 0.1 or less or 0.04 or more and 0.08 or less.

The intensity ratio range tends to increase with the increase in the stabilizing element amount and the increase in the M1 element amount.

Meanwhile, for the zirconia compositional substance in which the percentage of the measurement points at which the X-ray intensity ratio is 0.05 or more is 3% or less of all measurement points, the intensity ratio range may be any desired range; however, the percentage of the measurement points at which the X-ray intensity ratio is 0.05 or more is preferably 3% or less of all measurement points and the intensity ratio range is preferably 0.3 or less.

The maximum value (hereinafter may also be referred to as the "maximum intensity ratio") of the X-ray intensity ratio of the zirconia compositional substance of this embodiment is preferably 0.3 or less, 0.2 or less or 0.1 or less. When the maximum intensity ratio is in this range, the influence of the coarse aggregated particles of the M1 element on the thermal shrinkage behavior of the zirconia compositional substance tends to be smaller. The maximum intensity ratio of the zirconia compositional substance of this embodiment may be 0.04 or more or 0.05 or more. A preferable maximum intensity ratio is, for example,0.04 or more and 0.3 or less, 0.04 or more and 0.2 or less or 0.05 or more and 0.1 or less.

The minimum value (hereinafter may also be referred to as the "minimum intensity ratio") of the X-ray intensity ratio of the zirconia compositional substance of this embodiment is, for example, 0 or more, more than 0 or 0.001 or more, and may be 0.05 or less, 0.04 or less, 0.01, or less or 0.002 or less, for example, 0 or more and 0.01 or less, 0 or more and 0.002 or less or more than 0 and 0.002 or less.

For the zirconia compositional substance of this embodiment, the characteristic X-ray of the zirconium element and the characteristic X-ray of the M1 element may be measured with a field emission-wavelength-dispersive electron probe micro-analyzer (hereinafter may also be referred to as "FE-EPMA") under the following conditions.

Accelerating voltage: 15 kV
Irradiation current: 50 nA
Beam diameter: 1 $\mu$m
Capture time: 50 msec
Magnification: 500x
Analysis area: 256.0 $\mu$m $\times$ 256.0 $\mu$m

In the measurement, a common FE-EPMA apparatus (for example, JXA-iHP200F produced by JEOL Ltd.) may be used, and the analysis area may be divided into a 65,500 $\pm$ 100 grids in which each divided region may be used as a measurement point. In this embodiment, FE-EPMA measurement may be performed on multiple sites (preferably 5 $\pm$ 3 sites) of the same sample, the high-intensity percentage, the coarse intensity percentage, the maximum intensity ratio, the minimum intensity ratio and the intensity ratio range may be determined for each site, and the average thereof may be used as the values of this embodiment.

The zirconia compositional substance of this embodiment contains a coloring element (hereinafter may also be referred to as the "sub-color component") that is either or both of a lanthanoid rare earth element and a second transition metal element (hereinafter may also be referred to as the "M2 element") different from the M1 element. The sub-color component is a color component for adjusting the color tone, and the color tone of the sintered body to be obtained can be adjusted when the zirconia compositional substance contains a sub-color component in addition to the M1 element. Depending on the desired color tone, the sub-color component may be a lanthanoid rare earth element only, may be an M2 element only or may be a lanthanoid rare earth element and an M2 element.

A transition metal element serving as the M2 element is a transition metal element different from the M1 element contained in the compositional substance of this embodiment, has a function of coloring zirconia and is a transition metal element that is prone to segregate after sintering. The M2 element is more preferably at least one selected from the group consisting of manganese, iron, cobalt, nickel, copper, molybdenum, technetium, ruthenium, rhodium, palladium and silver and different from the transition metal element that serves as the M1 element. The M2 element is more preferably at least one selected from the group consisting of manganese, iron, cobalt, nickel, copper, molybdenum, technetium, ruthenium, rhodium, palladium and silver, more preferably at least one selected from the group consisting of manganese, iron, cobalt, nickel, copper, molybdenum and silver, yet more preferably at least one selected from the group consisting of manganese, iron, cobalt, nickel and copper, still more preferably at least one selected from the group consisting of manganese, cobalt and nickel, yet more preferably either or both of manganese and cobalt and most preferably cobalt.

The M2 element is preferably different from the M1 element and is manganese, iron, cobalt, nickel, copper, molybdenum or silver, is more preferably different from the M1 element and is manganese, iron, cobalt, is more preferably different from the M1 element and is manganese, iron, cobalt, nickel or copper, is yet more preferably different from the M1 element and is manganese, cobalt or nickel and is still more preferably different from the M1 element and is manganese or cobalt.

The content of the M2 element ("M2 element amount) is preferably less than 100 mass ppm, 80 mass ppm or less, 70 mass ppm or less of 50 mass ppm or less. At such a content, the influence of the M2 element on the thermal shrinkage behavior of the zirconia compositional substance is significantly reduced. The zirconia compositional substance of this embodiment may be free of the M2 element (in other words, the content of the M2 element may be 0 mass ppm). Thus, the M2 element amount in the zirconia compositional substance of this embodiment may be 0 mass ppm or more. Meanwhile, the zirconia compositional substance of this embodiment contains the M2 element, and the content thereof may be more than 0 mass ppm, 3 mass ppm or more, 10 mass ppm or more, 20 mass ppm or more or 35 mass ppm or more, for example, more than 0 mass ppm and less than 100 mass ppm, more than 0 mass ppm and 80 mass ppm or less or more than 0 mass ppm and 50 mass ppm or less. The M2 element amount may be more than 0 mass ppm and 10 mass ppm or less or more than 0 mass ppm and 5 mass ppm or less.

The M2 element amount in this embodiment is the mass ratio [mass ppm] of the M2 element in terms of oxide relative to the metal element mass.

The oxide of the transition metal element used in the conversion may be $Mn_3O_4$ for manganese, $Fe_2O_3$ for iron,

$Co_3O_4$ for cobalt, $NiO$ for nickel, $CuO$ for copper, $Mo_2O_3$ for molybdenum, $Tc_2O_3$ for technetium, $RuO_2$ for ruthenium, $RhO_2$ for rhodium, $Pd_2O_3$ for palladium and $Ag_2O$ for silver.

The lanthanoid rare earth element is preferably at least one selected from the group consisting of praseodymium (Pr), neodymium (Nd), samarium (Sm), europium (Eu), gadolinium (Gd), terbium (Tb), erbium (Er) and ytterbium (Yb), is more preferably at least one selected from the group consisting of praseodymium, neodymium, terbium and erbium, is yet more preferably at least one selected from the group consisting of praseodymium, terbium and erbium, is still more preferably either or both of terbium and erbium and is particularly preferably erbium.

The lanthanoid rare earth element is considered to have less influence on the thermal shrinkage behavior of the zirconia compositional substance than transition metal elements. Thus, the content of the lanthanoid rare earth element is preferably 1.0 mass% or less, 0.85 mass% or less, 0.6 mass% or less or 0.15 mass% or less. The zirconia compositional substance of this embodiment may be free of the lanthanoid rare earth element (in other words, the content of the lanthanoid rare earth element may be 0 mass% or 0 mass% or more). Meanwhile, the zirconia compositional substance of this embodiment contains the lanthanoid rare earth element, and the content thereof may be more than 0 mass%, 0.01 mass% or more or 0.05 mass% or more, for example, 0 mass% or more and 1.0 mass% or less, 0 mass% or more and 0.15 mass% or less or 0.05 mass% or more and 0.15 mass% or less. The lanthanoid rare earth element also functions as a stabilizing element. However, for the sake of convenience, the lanthanoid rare earth element is deemed to be not included in the stabilizing element in this embodiment.

The content of the lanthanoid rare earth element may be determined as the percentage [mass%] of the lanthanoid rare earth element [g] in terms of oxide relative to zirconia and the metal element mass [g].

The oxide of the lanthanoid rare earth element used in the conversion may be $Pr_6O_{11}$ for praseodymium, $Nd_2O_3$ for neodymium, $Sm_2O_3$ for samarium, $Eu_2O_3$ for europium, $Gd_2O_3$ for gadolinium, $Tb_4O_7$ for terbium, $Er_2O_3$ for erbium and $Yb_2O_3$ for ytterbium.

The zirconia compositional substance of this embodiment may contain the sub-color component in any form. The sub-color component contains, for example, at least one selected from the group consisting of an oxide, a hydroxide, an oxyhydroxide, a halide, a sulfate, a nitrate and an acetate, at least one selected from the group consisting of an oxide, a hydroxide, an oxyhydroxide and a chloride, either or both of an oxide and an oxyhydroxide or an oxide.

Since abnormal grain growth during sintering tends to be suppressed, when the sub-color component is a lanthanoid rare earth element, the lanthanoid rare earth element is preferably dissolved in zirconia (substituting zirconium cations). The zirconia compositional substance of this embodiment preferably contains zirconia in which a lanthanoid rare earth element is dissolved (hereinafter may also be referred to as the "lanthanoid-dissolved zirconia") instead of or in addition to the lanthanoid rare earth element. In particular, the zirconia compositional substance of this embodiment preferably contains a lanthanoid rare earth element only as the lanthanoid-dissolved zirconia, and more preferably does not contain a compound containing a lanthanoid rare earth element, such as a lanthanoid rare earth oxide.

In this embodiment, the absence of the compound containing the lanthanoid rare earth element may be confirmed by the absence of particulate lanthanoid rare earth elements under observation by FE-EPMA.

The zirconia compositional substance of this embodiment may contain at least one third transition metal element (hereinafter may also be referred to as the "M3 element") selected from the group consisting of titanium (Ti), vanadium(V) and niobium (Nb), preferably contains either or both of titanium and vanadium, and more preferably contains titanium. The M3 element is a transition metal element that rarely segregates after sintering. When the M3 element is contained in addition to or instead of the sub-color component, the changes in thermal shrinkage behavior of the zirconia compositional substance can be reduced and the color tone of the sintered body to be obtained can be finely adjusted.

The zirconia compositional substance of this embodiment contains the M3 element, and the content thereof may be 1.0 mass% or less, 0.5 mass% or less or 0.3 mass% or less, or is, for example, more than 0 mass% or 0.1 mass% or more. Alternatively, the zirconia compositional substance of this embodiment may be free of the M3 element (in other words, the content of the M3 element may be 0 mass% or 0 mass% or more). Examples of the content of the M3 element in this embodiment (the M3 element amount) include 0 mass% or more and 1.0 mass% or less, 0 mass% or more and 0.5 mass% or less and 0 mass% or more and 0.05 mass% or less; and when the M3 element is contained, the examples thereof include more than 0 mass% and 1.0 mass% or less, more than 0 mass% and 0.5 mass% or less and more than 0 mass% and 0.05 mass% or less.

The content of the M3 element may be determined as the mass percentage [mass%] of the M3 element [g] in terms of oxide relative to zirconia and the metal element mass [g]. The oxide used in conversion is $TiO_2$ for titanium, $V_2O_5$ for vanadium and $Nb_2O_3$ for niobium.

The zirconia compositional substance of this embodiment may contain the M3 element in any form. The M3 element contains, for example, at least one selected from the group consisting of an oxide, a hydroxide, an oxyhydroxide, a halide, a sulfate, a nitrate and an acetate, at least one selected from the group consisting of an oxide, a hydroxide, an oxyhydroxide and a chloride, either or both of an oxide and an oxyhydroxide or an oxide. Furthermore, the M3 element may be entirely or partly dissolved in zirconia (may substitute zirconium cations).

The zirconia compositional substance of this embodiment may contain at least one selected from the group

consisting of alumina ($Al_2O_3$), silica ($SiO_2$) and germania ($Ge_2O_3$) (hereinafter may also be referred to as the "additive component") and may further contain alumina. By containing minute amounts of additive components, densification at low temperature is easily accelerated. When additive components are contained, the content thereof may be more than 0 mass%, 0.005 mass% or more or 0.01 mass% or more and may be 0.25 mass% or less, 0.2 mass% or less, 0.1 mass% or less or 0.06 mass% or less. Alternatively, the zirconia compositional substance of this embodiment may be free of an additive component (in other words, the content of the additive component may be 0 mass% or 0 mass% or more).

The content of the additive component may be determined as a mass percentage [mass%] of the additive component ($Al_2O_3$, $SiO_2$ or $Ge_2O_3$) [g] relative to the metal element mass [g].

The zirconia compositional substance of this embodiment is preferably free of impurities but may contain hafnia ($HfO_2$), which is an inevitable impurity of zirconia. The content of hafnia as an inevitable impurity differs significantly depending on the raw material ore or the production method, and is, for example, 2.0 mass% or less. In calculating the values based on the composition, such as density, in this embodiment, hafnia may be deemed as zirconia ($ZrO_2$).

The zirconia compositional substance of this embodiment may contain a binder. By containing a binder, the operation ease (handling) and shape retaining properties are further enhanced. The binder may be any binder that can be used in granulation and forming of ceramics, and is preferably an organic binder. The organic binder is, for example, at least one selected from the group consisting of polyvinyl alcohol, polyvinyl butyrate, wax and acrylic resin, and is preferably either or both of polyvinyl alcohol and acrylic resin and more preferably acrylic resin. In this embodiment, the acrylic resin is a polymer that contains either or both of an acrylic acid ester and a methacrylic acid ester. A specific example of the binder is at least one selected from the group consisting of AS-1100, AS-1800 and AS-2000 (all trade names, produced by Toa Gosei Co., Ltd.).

The content of the binder is 0.5 mass% or more or 1 mass% or more or 10 mass% or less or 5 mass% or less, for example.

For example, the composition of an yttrium-stabilized zirconia compositional substance containing iron as the M1 element, cobalt as the M2 element, erbium as the lanthanoid rare earth element, titanium as the M3 element, an aluminum (alumina) as the additive component and a binder can be determined as follows.

Metal element mass [g]:

$$Y_2O_3 + Er_2O_3 + ZrO_2 + Al_2O_3 + Fe_2O_3 + Co_3O_4 + TiO_2$$

Stabilizing element amount [mol%]:

$$\{(Y_2O_3 + Er_2O_3)/(Y_2O_3 + Er_2O_3 + ZrO_2)\} \times 100$$

Erbium content [mass%]:

$$\{Er_2O_3/(Y_2O_3 + Er_2O_3 + ZrO_2 + Al_2O_3 + Fe_2O_3 + Co_3O_4 + TiO_2)\} \times 100$$

Iron content [mass%]:

$$\{Fe_2O_3/(Y_2O_3 + Er_2O_3 + ZrO_2 + Al_2O_3 + Fe_2O_3 + Co_3O_4 + TiO_2)\} \times 100$$

Cobalt content [mass%]:

$$\{Co_3O_4/(Y_2O_3 + Er_2O_3 + ZrO_2 + Al_2O_3 + Fe_2O_3 + Co_3O_4 + TiO_2)\} \times 100$$

Titanium content [mass%]:

$$\{TiO_2/(Y_2O_3 + Er_2O_3 + ZrO_2 + Al_2O_3 + Fe_2O_3 + Co_3O_4 + TiO_2)\} \times 100$$

Alumina content [mass%]:

$$\{Al_2O_3/(Y_2O_3 + Er_2O_3 + ZrO_2 + Al_2O_3 + Fe_2O_3 + Co_3O_4 + TiO_2)\} \times 100$$

In addition, the binder content may be determine as follows.

$$\{(W_1 - W_2)/W_1\} \times 100$$

In the formula above, $W_1$ represents the mass [g] of the zirconia compositional substance before the heat treatment in an air atmosphere at 250°C or higher and 400°C or lower, and $W_2$ represents the mass [g] of the zirconia compositional substance before the heat treatment in an air atmosphere at 250°C or higher and 400°C or lower. Since the calculation method is different, the apparent total content of the zirconia compositional substance containing the binder does not have to be 100 mass%.

The BET specific surface area of the zirconia compositional substance of this embodiment is, for example, 8 $m^2/g$ or more or 10 $m^2/g$ or more and 15 $m^2/g$ or less, 11 $m^2/g$ or less; or 10.5 $m^2/g$ or less; and is preferably 8 $m^2/g$ or more and 15 $m^2/g$ or less, 10 $m^2/g$ or more and 11 $m^2/g$ or less or 10 $m^2/g$ or more and 10.5 $m^2/g$ or less.

The average particle size of the zirconia compositional substance of this embodiment is 0.35 $\mu$m or more or 0.4 $\mu$m or more and 0.55 $\mu$m or less or 0.5 $\mu$m or less, for example, and is preferably 0.35 $\mu$m or more and 0.55 $\mu$m or less or 0.4 $\mu$m or more and 0.5 $\mu$m or less.

The T + C phase ratio of the zirconia compositional substance of this embodiment is 50% or more, 55% or more, 60% or more, 70% or more, 80% or more or 85% or more, and 100% or less, 99% or less, 95% or less, 92% or less or 90% or less, for example, and is preferably 50% or more and 100% or less, 70% or more and 95% or less or 85% or more and 92% or less.

When the zirconia compositional substance of this embodiment is a powder (hereinafter may also be referred to as the "powder of this embodiment"), the powder may be a granular powder. In this manner, the flowability and the formability tend to be high.

The average granule size of the granular powder is 30 $\mu$m or more or 40 $\mu$m or more and 100 $\mu$m or less or 50 $\mu$m or less, for example, and is preferably 40 $\mu$m or more and 50 $\mu$m or less.

The bulk density of the powder of this embodiment is, for example, 1.10 $g/cm^3$ or more, 1.15 $g/cm^3$ or more or 1.20 $g/cm^3$ or more and 1.40 $g/cm^3$ or less, 1.35 $g/cm^3$ or less or 1.30 $g/cm^3$ or less, and is preferably 1.15 $g/cm^3$ or more and 1.40 $g/cm^3$ or less or 1.20 $g/cm^3$ or more and 1.30 $g/cm^3$ or less.

The density of the zirconia powder of this embodiment after being filled in a mold having a diameter of 25 mm, uniaxially press-formed at a pressure of 49 MPa, and subjected to a CIP process at a pressure of 196 MPa may be 3.2 $g/cm^3$ or more or 3.3 $g/cm^3$ or more and 3.5 $g/cm^3$ or less or 3.4 $g/cm^3$ or less. In particular, the measured density of the zirconia powder of this embodiment after $3.0 \pm 0.1$ g of the zirconia powder is filled in a mold having a diameter of 25 mm, uniaxially press-formed at a pressure of 49 MPa, and subjected to a CIP process at a pressure of 196 MPa is, for example, 3.2 $g/cm^3$ or more or 3.3 $g/cm^3$ or more and 3.5 $g/cm^3$ or less or 3.4 $g/cm^3$ or less, and is preferably 3.2 $g/cm^3$ or more and 3.5 $g/cm^3$ or less or 3.3 $g/cm^3$ or more and 3.4 $g/cm^3$ or less.

In addition, when the zirconia compositional substance of this embodiment is a green body (hereinafter may also be referred to as the "green body of this embodiment"), the measured density thereof may be the same as the aforementioned measured density, is, for example, 3.2 $g/cm^3$ or more or 3.3 $g/cm^3$ or more and 3.5 $g/cm^3$ or less or 3.4 $g/cm^3$ or less; and is preferably 3.2 $g/cm^3$ or more and 3.5 $g/cm^3$ or less or 3.3 $g/cm^3$ or more and 3.4 $g/cm^3$ or less.

The shape of the green body of this embodiment may be the same as the shape of the target calcined body, and is, for example, at least one selected from the group consisting of a disk shape, a columnar shape, a cubic shape, a cuboid shape, a polyhedral shape, a conical shape, a spherical shape and a substantially spherical shape or any shape such as a shape of a dental prosthesis.

The green body of this embodiment may have a hardness that is resistant breaking during handling, for example, Vickers hardness of 10.0 HV or more, 11.0 or more or 11.5 HV or more and 14.0 HV or less, 13.0 HV or less or 12.0 HV or less, or 10.0 HV or more and 14.0 HV or less, 11.0 HV or more and 13.0 HV or less or 11.5 HV or more and 12.0 HV or less. When the Vickers hardness is 11.0 or more, defects during handling are further inhibited.

The green body of this embodiment is obtained by forming a powder of this embodiment, but the values, such as high-intensity percentage, measured by FE-EPMA, the composition, the BET specific surface area etc., do not change between before and after forming.

The zirconia compositional substance of this embodiment can be used in known usage of zirconia but is preferably used as a precursor of either or both of a sintered body and a calcined body and is preferably used as either or both of a dental material or a precursor thereof.

[Method for producing zirconia compositional substance]

The zirconia compositional substance of this embodiment may be produced by any method from which a zirconia compositional substance that satisfies the aforementioned features is obtained.

An example of a preferable method for producing a zirconia compositional substance of this embodiment in a powder form is a method for producing a zirconia compositional substance (zirconia powder), the method including a mixing step of mixing a zirconia powder containing at least one M1 element selected from the group consisting of manganese, iron, cobalt, nickel, copper, molybdenum, technetium, ruthenium, rhodium, palladium and silver and stabilizing element-containing zirconia, in which, when the characteristic X-ray of the zirconium element and the characteristic X-ray of the M1 element are measured, the percentage of measurement points at which the ratio of the intensity of the characteristic X-ray of the M1 element to the intensity of the characteristic X-ray of the zirconium element is 0.05 or more is 2% or less of all measurement points, and a zirconia powder containing zirconia and a coloring element that is either or both of a lanthanoid rare earth element and an M2 element different from the M1 element such that the content of the M1 element is 100 mass ppm or more and the content of the M2 element is less than 100 mass ppm.

In the mixing step, a zirconia powder containing at least one M1 element selected from the group consisting of manganese, iron, cobalt, nickel, copper, molybdenum, technetium, ruthenium, rhodium, palladium and silver and stabilizing element-containing zirconia, in which, when the characteristic X-ray of the zirconium element and the characteristic X-ray of the M1 element are measured, the percentage of measurement points at which the ratio of the intensity of the characteristic X-ray of the M1 element to the intensity of the characteristic X-ray of the zirconium element is 0.05 or more is 2% or less of all measurement points (hereinafter this powder may also be referred to as the "raw material powder 1") is mixed with a zirconia powder containing zirconia and a coloring element that is either or both of a lanthanoid rare earth element and an M2 element different from the M1 element (hereinafter this powder may also be referred to as the "raw material powder 2"). Unlike the case in which a zirconia powder and compounds of the M1 element and the like are directly mixed to obtain a mixed powder, zirconia that already contains the M1 element etc., is used as a starting substance in the production method of this embodiment. In this manner, the M1 element and the coloring element tend to be more homogeneous even by a dry mixing and other simple mixing methods.

The raw material powder 1 contains the M1 element. The M1 element contained in the raw material powder 1 may be the same as the M1 element in the zirconia compositional substance of this embodiment.

In order to adjust the content of the M1 element in the zirconia compositional substance obtained in the mixing step to 100 mass ppm or more, the content of the M1 in the raw material powder 1 may be the same as the M1 element amount in the zirconia compositional substance of the aforementioned embodiment; for example, the content of the M1 element is more than 0.01 mass% (more than 100 mass ppm), 0.05 mass% or more (500 mass ppm or more) or 0.1 mass% or more (1000 mass ppm or more) and 0.3 mass% or less (3000 mass ppm or more), 0.25 mass% or less (2500 mass ppm or less) or 0.2 mass% or less (2000 mass ppm or less).

According to the raw material powder 1, the percentage (high-intensity percentage) of measurement points at which, when the characteristic X-ray of the zirconium element and the characteristic X-ray of the M1 element are measured, the ratio (X-ray intensity ratio) of the intensity of the characteristic X-ray of the M1 element to the intensity of the characteristic X-ray of the zirconium element is 0.05 or more is 2% or less of all measurement points. Presumably, aggregation of the M1 element in the raw material powder 1 is reduced by satisfying such a high-intensity percentage.

The high-intensity percentage in the raw material powder 1 is, for example, 2% or less, 1% or less or 0.1% or less and 0% or more, more than 0% or 0.01% or more, or 0% or more and 2% or less, more than 0% and 1% or less or 0.01% or more and 0.1% or less.

The raw material powder 1 is preferably free of coarse aggregation of the M1 element, and the percentage of the measurement points at which the X-ray intensity ratio is 0.1 or more ("coarse intensity percentage") is preferably 0.5% or less, 0.1% or less or 0.05% or less. The coarse intensity percentage may be 0% or more or more than 0%, and is preferably 0% or more and 0.5% or less or 0% or more and 0.05% or less.

The characteristic X-ray of the zirconium element and the characteristic X-ray of the M1 element of the raw material powder 1 of this embodiment may be measured by FE-EPMA as with the zirconia compositional substance of this embodiment except that the following conditions are employed.

Accelerating voltage: 15 kV
Irradiation current: 50 nA
Beam diameter: 1 $\mu$m
Capture time: 50 msec
Magnification: 5000x
Analysis area: 45.32 $\mu$m $\times$ 45.32 $\mu$m

The maximum value ("maximum intensity ratio") of the X-ray intensity ratio of the raw material powder 1 is preferably 0.9 or less, 0.5 or less or 0.2 or less. When the X-ray intensity ratio is in this range, aggregation of the M1 element in the zirconia compositional substance of this embodiment is inhibited. The maximum intensity ratio of the raw material powder 1 may be 0.05 or more or 0.08 or more and is, for example, 0.05 or more and 0.9 or less or 0.08 or more and 0.2 or less.

The minimum intensity ratio of the X-ray intensity ratio of the raw material powder 1 is, for example, 0 or more or more than 0, and may be 0.01 or less, 0 or more and 0.01 or less or more than 0 and 0.01 or less.

For the same reason as the intensity ratio range of the zirconia compositional substance of this embodiment, the distribution width (intensity ratio range) of the X-ray intensity ratio of the raw material powder 1 is 0.9 or less, 0.5 or less or 0.2 or less and may be more than 0 or 0.01 or more, more than 0 and 0.9 or less or 0.01 or more and 0.2 or less, for example.

The raw material powder 1 contains stabilizing element-containing zirconia. The type and content of the stabilizing element may be the same as the stabilizing element-containing zirconia contained in the zirconia compositional substance of this embodiment.

The raw material powder 2 contains a coloring element (sub-color component) that is either or both of a lanthanoid rare earth element and a M2 element different from the M1 element. The preferable sub-color component contained in the raw material powder 2 may be the same as that in the zirconia compositional substance of this embodiment.

The content of the M2 element in the raw material powder 2 may be a value similar to the M2 element amount in the zirconia compositional substance of this embodiment described above, and is, for example, 800 mass ppm or less, 500 mass ppm or less or 100 mass ppm or less and more than 0 mass ppm, 10 mass ppm or more or 30 mass ppm or more, for example.

The preferable content of the lanthanoid rare earth element contained in the raw material powder 2 may be a value similar to the lanthanoid rare earth element content in the zirconia compositional substance of this embodiment described above, and is, for example, 4 mass% or more, 6 mass% or more or 8 mass% or more and 20 mass% or less, 15 mass% or less, 12 mass% or less or 10 mass% or less.

The raw material powder 2 contains zirconia. When the sub-color component is the M2 element, the zirconia is preferably stabilizing element-containing zirconia. In contrast, when the sub-color component is a lanthanoid rare earth element, the zirconia is preferably lanthanoid rare earth element-dissolved zirconia.

When the zirconia contained in the raw material powder 2 is stabilizing element-containing zirconia, the type and content of the stabilizing element may be the same as those of the stabilizing element-containing zirconia contained in the zirconia compositional substance of this embodiment.

When the zirconia contained in the raw material powder 2 is stabilizing element-containing zirconia, the stabilizing element amount is preferably about the same between the raw material powders 1 and 2; for example, the difference in the stabilizing element amount between the raw material powders 1 and 2 is 1.5 mol% or less or 1.0 mol% or less and 0 mol% or more or more than 0 mol%. Meanwhile, when the raw material powder 2 contains a lanthanoid rare earth element, the difference in the stabilizing element amount between the raw material powders 1 and 2 is equal to the stabilizing element amount of the raw material powder 1.

To achieve the target contents of the M1 element and the coloring element, in the mixing step, a powder of stabilizing element-containing zirconia free of transition metal elements and lanthanoid rare earth elements (hereinafter this powder may also be referred to as the "raw material powder 3") is preferably mixed in addition to the raw material powder 1 and the raw material powder 2.

The raw material powder 3 is stabilizing element-containing zirconia powder free of transition metal elements and lanthanoid rare earth elements and is preferably an yttrium-containing zirconia powder free of transition metal elements and lanthanoid rare earth elements.

The stabilizing element amount in the raw material powder 3 may be the same as the type and content of the stabilizing element in the raw material powder 1 and the zirconia compositional substance of this embodiment.

In the mixing step, an M3 element source may be used in addition to the raw material powders 1 to 3. Examples of the M3 element source include either or both of a salt and a compound that contain at least one selected from the group consisting of titanium, vanadium and niobium; for example, the M3 element source is at least one selected from the group consisting of an oxide, a hydroxide, an oxyhydroxide, a halide, a carbonate, a sulfate, a nitrate and an acetate that contain at least one selected from the group consisting of titanium, vanadium and niobium, is preferably at least one selected from the group consisting of an oxide, a hydroxide, an oxyhydroxide, a chloride and an acetate that contain at least one selected from the group consisting of titanium, vanadium and niobium, and is more preferably either or both of an oxide and a hydroxide that contain at least one selected from the group consisting of titanium, vanadium and niobium.

The titanium source is, for example, at least one selected from the group consisting of titanium oxide ($TiO_2$), titanium hydroxide ($Ti(OH)_2$), titanium oxyhydroxide ($TiOOH$), titanium(II) chloride ($TiCl_2$), titanium (III) chloride ($TiCl_3$), titanium(IV) chloride ($TiCl_4$), titanium sulfate ($Ti(SO_4)_2$), titanium nitrate ($Ti(NO_3)_2$) and titanium acetate ($Ti(CH_3COO)_4$).

The vanadium source is, for example, at least one selected from the group consisting of vanadium oxide ($VO$), vanadium pentaoxide ($V_2O_5$), vanadium hydroxide ($V(OH)_2$), vanadium oxyhydroxide ($VO(OH)_2$), vanadium(III) chloride ($VCl_3$), vanadium sulfate ($V_2(SO_4)_3$) and vanadium acetate ($V(CH_3COO)_4$).

The niobium source is, for example, at least one selected from the group consisting of niobium oxide ($Nb_2O_5$), vanadium hydroxide ($V(OH)s$), niobium(V) chloride ($Nbls$) and niobium acetate ($V(CH_3COO)_3$).

The M3 element source may be mixed such that the zirconia compositional substance obtained by the mixing step has an M3 element content equal to that of the zirconia compositional substance of the aforementioned embodiment.

Moreover, at least one of the raw material powders 1 to 3 may contain the M3 element in addition to or instead of providing the M3 element source.

In the mixing step, an additive component source may be used in addition to the raw material powders 1 to 3.

The additive component source is, for example, either or both of a salt and a compound that contain at least one selected from the group consisting of aluminum, silicon and germanium, and an example thereof is at least one selected from the group consisting of an oxide, a hydroxide, an oxyhydroxide, a halide, a carbonate, a sulfate, a nitrate and an acetate that contain at least one selected from the group consisting of aluminum, silicon and germanium. The additive component source is preferably at least one selected from the group consisting of alumina, silica and germania and is further preferably alumina, for example.

The additive component source may be mixed such that the zirconia compositional substance obtained by the mixing step has an additive component content equal to that of the zirconia compositional substance of the aforementioned embodiment.

Moreover, at least one of the raw material powders 1 to 3 may contain the additive component in addition to or instead of providing the additive component source.

The raw material powders 1 to 3 may contain a binder. By containing a binder, the operation ease (handling) and shape retaining properties are further enhanced. The binder and the content thereof may be the same as the binder contained in the zirconia compositional substance of this embodiment.

Each of the starting substances such as the raw material powders 1 to 3 preferably has physical properties similar to the zirconia powder of this embodiment, and preferably has physical properties similar to each other. Examples of the physical properties of each starting substance are as follows.

The BET specific surface area of each starting substance is, for example, preferably 8 m$^2$/g or more or 10 m$^2$/g or more and 15 m$^2$/g or less, 12 m$^2$/g or less, 11 m$^2$/g or less or 10.5 m$^2$/g or less, for example, 8 m$^2$/g or more and 15 m$^2$/g or less or 10 m$^2$/g or more and 12 m$^2$/g or less.

The average particle size of each starting substance is preferably 0.35 $\mu$m or more or 0.4 $\mu$m or more and 0.55 $\mu$m or less or 0.5 $\mu$m or less, for example, 0.35 $\mu$m or more and 0.55 $\mu$m or less or 0.4 $\mu$m or more and 0.5 $\mu$m or less.

The T + C phase ratio of each starting substance is preferably 50% or more, 55% or more or 60% or more and 100% or less, 99% or less, 95% or less, 92% or less or 90% or less.

Each starting substance is preferably a granular powder. The average granule size of the granular powder is 30 $\mu$m or more or 40 $\mu$m or more and 100 $\mu$m or less or 50 $\mu$m or less, for example, and is preferably 30 $\mu$m or more and 100 $\mu$m or less or 40 $\mu$m or more and 50 $\mu$m or less.

In the mixing step, the starting substances may be mixed such that the content of the M1 element is 100 mass ppm or more and the content of the M2 element is less than 100 mass ppm and that the these contents are equal to the contents of the M1 and the coloring element in the zirconia compositional substance of the aforementioned embodiment.

The mixing method may be any desired mixing method that can mix the starting substances to homogeneity, may be at least one of dry mixing and wet mixing and may be dry mixing. In the production method of this embodiment, since zirconia powders already containing the M1 element and the color component are mixed, localization of the M1 element rarely occurs even by a simple mixing method such as shaking or stirring mixing.

The mixing percentages of the starting substances may be any mixing percentages in conformity with the target content of the M1 element etc., and the target color tone. For example, the starting substances may be mixed at the following percentages so that the total is 100 mass%.

Raw material powder 1: 15 mass% or more, 18 mass% or more or 20 mass% or more and 80 mass% or less, 75 mass% or less or 70 mass% or less.

Raw material powder 2 containing M2 element:

0 mass% or more, more than 0 mass%, 1 mass% or more, 3 mass% or more or 5 mass% or more and 30 mass% or less, 20 mass% or less or 18 mass% or less.

Raw material powder 2 containing lanthanoid rare earth element:

0 mass% or more, more than 0 mass%, 0.5 mass% or more, 0.8 mass% or more or 1 mass% or more and 10 mass% or less, 8 mass% or less or 3 mass% or less.

Raw material powder 3: 15 mass% or more, 18 mass% or more or 20 mass% or more and 80 mass% or less, 75 mass% or less or 70 mass% or less.

M3 element source: 0 mass% or more, more than 0 mass%, 0.05 mass% or more, 0.10 mass% or more or 0.20 mass% or more and 1.0 mass% or less, 0.8 mass% or less or 0.7 mass% or less.

Additive component source: 0 mass% or more, more than 0 mass%, 0.003 mass% or more or 0.01 mass% or more and

0.1 mass% or less, 0.08 mass% or less or 0.06 mass% or less.

When the zirconia compositional substance of this embodiment is a green body (compact), a preferable production method is a method for producing a zirconia compositional substance (zirconia green body), the method including a forming step of forming a zirconia powder obtained by the aforementioned mixing step.

The forming method may be any desired forming method that can prepare a compact of the zirconia compositional substance (zirconia powder) and is, for example, at least one method selected from the group consisting of uniaxial press forming, a cold isostatic pressing (hereinafter may also be referred to as "CIP") process, slip casting, sheet forming, drain casting and injection forming, is preferably at least one selected from the group consisting of slip casting, injection forming, uniaxial press forming and the CIP process, is more preferably either or both of uniaxial press forming and the CIP process, and is yet more preferably a method that involves uniaxially press-forming a zirconia compositional substance and subjecting the primary green body obtained as a result to a CIP process. The pressure in the uniaxial press forming is 15 MPa or more or 90 MPa or more and 400 MPa or less or 150 MPa or less, for example.

[Method for producing raw material powder]

An example of a preferable method for producing a raw material powder 1 is a production method that has a step of drying a slurry containing hydrated zirconia, a stabilizing element source and an M1 element source to obtain a dry powder and a heat treatment step of heat-treating the dry powder at a temperature lower than the sintering temperature. When powdery zirconia and an M1 element source are mixed, localization of the M1 element is likely to occur. The localized M1 element, even at the detection limit or lower, has a large influence on the thermal shrinkage behavior of the zirconia compositional substance to be obtained. In contrast, presumably since zirconia and the M1 element source are mixed at the stage of the precursor of the powder, localization of the M1 element that can influence the shrinkage behavior of the zirconia compositional substance is reduced. The raw material powder 1 is preferably a powder obtained from a solution containing a precursor of zirconia and an M1 element, a powder the precursor of which is either or both of a hydrolysis product and a coprecipitate obtained from a solution containing hydrated zirconia and an M1 element, or a powder the precursor of which is a hydrolysis product obtained from a solution containing hydrated zirconia and an M1 element.

In the step of drying a solution containing hydrated zirconia, a stabilizing element source and an M1 element source to obtain a dry powder (hereinafter this step may also be referred to as the "precursor synthesizing step"), a solution containing hydrated zirconia, a stabilizing element source and an M1 element source (hereinafter this solution may be referred to as the "raw material solution") is used. The contents of the hydrated zirconia, the stabilizing element source, and the M1 element source in the raw material solution may be the amounts similar to those of the composition of the aforementioned raw material powder 1.

The pH of the raw material solution for promoting dispersing of hydrated zirconia is preferably 7 or less or 5 or less and 1 or more or 3 or more.

Hydrated zirconia is zirconia in a hydrated state ($ZrO_2 \cdot nH_2O$, where n represents an integer), and a hydrated zirconia sol is more preferable. The hydrated zirconia is preferably obtained by at least one selected from the group consisting of hydrolysis, coprecipitation and neutralization of a zirconium salt, is preferably obtained by hydrolysis, is preferably obtained by hydrolysis or coprecipitation of at least one selected from the group consisting of zirconium oxychloride, zirconium oxynitrate dihydrate, zirconium chloride and zirconium sulfate and is preferably obtained by hydrolysis or coprecipitation of zirconium oxychloride.

The stabilizing element source (hereinafter, when yttrium or the like is a stabilizing element, this is referred to as the "yttrium source" or the like) may be either or both of a salt and a compound that contain a stabilizing element, for example, at least one selected from the group consisting of an oxide, a hydroxide, an oxyhydroxide, a halide, a carbonate, a sulfate, a nitrate and an acetate that contain a stabilizing element, is more preferably at least one selected from an oxide, a hydroxide and a chloride that contain a stabilizing element, and is further preferably a chloride containing a stabilizing element.

Examples of the yttrium source include at least one selected from the group consisting of yttrium oxide, yttrium hydroxide, yttrium oxyhydroxide, yttrium chloride, yttrium carbonate, yttrium sulfate, yttrium nitrate and yttrium acetate, at least one selected from the group consisting of yttrium oxide, yttrium hydroxide, yttrium oxyhydroxide and yttrium chloride, and either or both of yttrium oxide and yttrium chloride, and the yttrium source is preferably yttrium chloride.

Examples of the calcium source include at least one selected from the group consisting of calcium oxide, calcium hydroxide, calcium oxyhydroxide, calcium chloride, calcium carbonate, calcium sulfate, calcium nitrate and calcium acetate, at least one selected from the group consisting of calcium oxide, calcium hydroxide, calcium oxyhydroxide and calcium chloride, and either or both of calcium oxide and calcium chloride.

Examples of the magnesium source include at least one selected from the group consisting of magnesium oxide, magnesium hydroxide, magnesium oxyhydroxide, magnesium chloride, magnesium carbonate, magnesium sulfate, magnesium nitrate and magnesium acetate, at least one selected from the group consisting of magnesium oxide, magnesium hydroxide, magnesium oxyhydroxide and magnesium chloride, and either or both of magnesium oxide and magnesium chloride.

The M1 element source (hereinafter, when the M1 element is iron or the like, this is referred to as the "iron source" or the like) may be either or both of a salt and a compound that contain an M1 element, is, for example, at least one selected from the group consisting of an oxide, a hydroxide, an oxyhydroxide, a halide, a carbonate, a sulfate, a nitrate and an acetate that contain an M1 element, is more preferably at least one selected from an oxide, a hydroxide and a chloride that contain an M1 element, and is further preferably an oxide that contains an M1 element. Examples of the main M1 source are as follows.

Examples of the manganese source include at least one selected from the group consisting of manganese(II) oxide (MnO), manganese(III) oxide (Mn$_2$O$_3$), manganese dioxide (MnO$_2$), trimanganese tetroxide (Mn$_3$O$_4$), manganese hydroxide (Mn(OH)$_2$), manganese oxyhydroxide (MnOOH), manganese chloride (MnCl$_2$), manganese carbonate (MnCO$_3$), manganese sulfate (MnSO$_4$), manganese nitrate (Mn(NO$_3$)$_2$) and manganese acetate (Mn(COOH)$_2$) and either or both of manganese hydroxide and manganese acetate.

Examples of the iron source include at least one selected from the group consisting of iron(II) oxide (FeO), iron(III) oxide (Fe$_2$O$_3$), triiron tetroxide (Fe$_3$O$_4$), iron(II) hydroxide (Fe(OH)$_2$), iron(III) hydroxide (Fe(OH)$_3$), iron(II) chloride (FeCl$_2$), iron(III) chloride (FeCl$_3$) and iron carbonate (FeCO$_3$), and the iron source is preferably at least one selected from the group consisting of iron(III) hydroxide, iron(II) hydroxide, iron(III) chloride and iron(II) chloride.

An example of the cobalt source is at least one selected from the group consisting of cobalt(II) oxide (CoO), cobalt(IV) oxide (CoO$_2$), tricobalt tetra oxide (Co$_3$O$_4$), cobalt hydroxide (Co(OH)$_2$), cobalt oxyhydroxide (CoOOH), cobalt chloride (CoCl$_2$), cobalt carbonate (CoCO$_3$), cobalt sulfate (CoSO$_4$), cobalt nitrate (Co(NO$_3$)$_2$) and cobalt acetate (Co(CH$_3$OO)$_2$).

An example of the nickel source is at least one selected from the group consisting of nickel(II) oxide (NiO), nickel(IV) oxide (NiO$_2$), trinickel tetraoxide (Ni$_3$O$_4$), nickel hydroxide (Ni(OH)$_2$), nickel oxyhydroxide (NiOOH), nickel chloride (NiCl$_2$), nickel carbonate (NiCO$_3$), nickel sulfate (NiSO$_4$), nickel nitrate (Ni(NO$_3$)$_2$) and nickel acetate (Ni(CH$_3$COO)$_2$).

An example of the copper source is at least one selected from the group consisting of copper(II) oxide (CuO), copper(IV) oxide (CuO$_2$), tricopper tetraoxide (Cu$_3$O$_4$), copper hydroxide (Cu(OH)$_2$), copper oxyhydroxide (CuOOH), copper chloride (CuCl$_2$), copper carbonate (CuCO$_3$), copper sulfate (CuSO$_4$), copper nitrate (Cu(NO$_3$)$_2$) and copper acetate (Cu(CH$_3$COO)$_2$).

The solvent contained in the raw material solution may be any solvent in which hydrated zirconia disperses, may be either or both of a polar solvent and an apolar solvent, is preferably either or both of alcohol and water, is more preferably either or both of ethanol and water, and is still more preferably water. An example of water contained in the raw material compositional substance is either or both of pure water and ion exchange water. In addition, a solution containing hydrated zirconia obtained by at least one selected from the group consisting of hydrolysis, coprecipitation and neutralization of a zirconium salt and a solvent therefor may be used as the hydrated zirconia and the solvent.

When the raw material powder 1 contains the M3 element, the raw material solution may contain the aforementioned M3 element source.

The raw material solution can be obtained by any method as long as hydrated zirconia, the stabilizing element source and the M1 element source are mixed to homogeneity. Examples of the method for producing the raw material solution include (1) a method that involves mixing hydrated zirconia, a stabilizing element source, an M1 element source and a solvent, (2) a method that involves mixing a hydrated zirconia solution, a stabilizing element source and an M1 element source and (3) a method that involves mixing a hydrated zirconia solution, a solution containing a stabilizing element source and a solution containing an M1 element source. Examples of the preferable mixing method include a production method that includes a step of mixing a hydrated zirconia solution, a solution containing an M1 element source and a stabilizing element source and a method that includes a step of mixing a hydrated zirconia solution obtained by hydrolysis of a zirconium salt, a solution containing an M1 element source and a stabilizing element source.

The drying method in the drying step may be any known method that removes the solvent and the hydration water of hydrated zirconia from the raw material solution. As a result, a dry powder is obtained. Any desired drying method may be selected as the drying method according to the concentration and the amount of the raw material solution used in the drying step. Examples of the drying conditions are as follows.

Drying atmosphere: air atmosphere, preferably, air-circulating atmosphere

Drying temperature: 150°C or higher, 160°C or higher or 180°C or higher and
210°C or lower, 200°C or lower or 190°C or lower

The drying method and the drying time for the raw material solution may be appropriately set according to the amount of the raw material solution to be processed and the type and the properties of the drying furnace. The drying time is, for example, 5 hours or more or 10 hours or more and 75 hours or less or 50 hours or less.

The method for producing the raw material powder 1 includes a heat treatment step of heat-treating the dry powder at a temperature lower than the sintering temperature. By this heat treatment, the stabilizing element is efficiently dissolved in zirconia and a raw material powder 1 is obtained. In addition, due to such a heat history prior to being formed into a green body (compact), aggregation of the M1 element in the heat treatment after forming is further reduced.

The heat treatment temperature in the heat treatment may be any desired temperature lower than the sintering temperature as long as dissolution of the stabilizing element into zirconia is accelerated and the desired BET specific surface area is achieved. The higher the heat treatment temperature, the lower the BET specific surface area tends to be. Examples of the heat treatment temperature include 1200°C or lower, lower than 1200°C or 1150°C or lower. The heat treatment temperature is preferably 1000°C or higher, 1050°C or higher or 1100°C or higher since dissolution of the stabilizing element into zirconia is further accelerated.

The heat treatment conditions other than the heat treatment temperature may be any conditions under which dissolution of the stabilizing element into zirconia is accelerated, for example, the following conditions.

Heat treatment atmosphere: oxidizing atmosphere, preferably, air atmosphere

Heat treatment temperature: 1000°C or higher, 1025°C or higher or 1050°C or higher and
1200°C or lower or 1150°C or lower

The heat treatment time may be appropriately adjusted according to the amount of the dry powder subjected to the heat treatment and the type and properties of the heat treatment furnace to be used and is, for example, 30 minutes or more or 1 hour or more and 10 hours or less or 5 hours or less.

When the raw material powder 1 is to contain an additive component, the aforementioned additive component may be added to the heat-treated raw material powder 1. The mixing method may be any known method and may be performed simultaneously with a grinding step described below. The amount of the additive component source contained in the raw material powder 1 may be the same as the aforementioned additive component amount.

In order to adjust the particle size of the raw material powder 1, the method for producing the raw material powder 1 may include a step of grinding the powder (hereinafter may also be referred to as the "grinding step"). Any method with which the powder achieves the desired particle size can be used as appropriate for grinding, and either or both of dry grinding and wet grinding may be employed. For high grinding efficiency, grinding is preferably wet grinding, grinding that uses at least one selected from the group consisting of a vibrating mill, a ball mill and a bead mill or grinding that uses a ball mill and a bead mill.

The grinding time may be appropriately set according to the amount of the calcined powder subjected to the grinding step and the grinding method. The longer the grinding time, the smaller the particle size will become until equilibrium is reached.

In order to control the flowability or improve the formability of the powder, the method for producing the raw material powder 1 may include a step of obtaining a granular powder by granulating the powder (hereinafter this step may also be referred to as the "granulating step"). Granulation may be performed by any desired method by which secondary particles of the powder undergo slow aggregation and turn into granular particles. The granulation method is, for example, at least one selected from the group consisting of a spray drying method, a stirring granulating method and an extrusion granulating method or is a spray drying method. In the spray drying method, a slurry containing a powder to be granulated dispersed in a solvent is formed into droplets and spray-dried to obtain a granular powder. The solvent may be either or both of water and alcohol. In addition, if necessary, a binder such as an acrylic resin may be mixed with the slurry and then spray drying may be performed to prepare granules.

The methods for producing the raw material powder 2 and the raw material powder 3 may be any desired methods.

For example, the raw material powder 2 containing the M2 element source is produced by the same method as the method for producing the raw material powder 1 except that an M2 element source is used instead of the M1 element source or by a production method that includes mixing a zirconia powder free of a color component and either or both of a compound and a salt that contain the M2 element.

In addition, for example, the raw material powder 2 containing a lanthanoid rare earth element is produced by the same method as the method for producing the raw material powder 1 except that a lanthanoid rare earth element source is used instead of the stabilizing element source and the M1 element source.

The lanthanoid rare earth element source may be either or both of a salt and a compound that contain a lanthanoid rare earth element, is, for example, at least one selected from the group consisting of an oxide, a hydroxide, an oxyhydroxide, a halide, a carbonate, a sulfate, a nitrate and an acetate that contain a lanthanoid rare earth element, is more preferably at least one selected from an oxide, a hydroxide and a chloride that contain a lanthanoid rare earth element, and is yet more preferably an oxide containing a lanthanoid rare earth element.

In addition, for example, the raw material powder 3 is produced by the same method as the method for producing the raw material powder 1 except that the M1 element is not used.

[Calcined body and production method therefor]

The zirconia compositional substance of this embodiment can be used as a precursor of a calcined body, and the zirconia compositional substance of this embodiment can be used in a method for producing a calcined body, the method including a step of calcining the zirconia compositional substance of this embodiment (hereinafter this step may be referred to as the "calcining step").

A calcined body (hereinafter may also be referred to as the "calcined body of this embodiment") is obtained by calcining the zirconia compositional substance.

Calcining in the calcining step may involve heat treatment at a temperature lower than the sintering temperature of zirconia and preferably involves pressureless firing. The calcining method may be any desired method as long as a calcined body having desired properties is obtained, and examples thereof are the following methods and conditions.

Calcining atmosphere: atmosphere other than reducing atmosphere, preferably oxidizing atmosphere and more preferably air atmosphere

Calcining temperature: 800°C or higher, 900°C or higher or 950°C or higher and

1200°C or lower, 1150°C or lower or 1100°C or lower

Heating rate: 10°C/hour or more or 30°C/hour or more and

200°C/hour or less or 150°C/hour or less

The calcining temperature retention time (hereinafter may also be referred to as the "calcining time") may be appropriately adjusted according to the shape, size and amount of the zirconia compositional substance subjected to calcining and the type and performance of a calcining furnace. The calcining time is, for example, 0.5 hours or more or 1 hour or more and may be 7 hours or less or 3 hours or less.

When the zirconia compositional substance of this embodiment contains a binder, the binder may be removed during heating to the calcining temperature. Meanwhile, prior to the calcining step, a step of removing the binder, that is, a debinding step, may be provided. The method for removing the binder may be any and may be appropriately adjusted according to the shape and size of the zirconia compositional substance and the type and properties of debinding, and an example thereof is heat treatment at 400°C or higher and lower than 800°C in an air atmosphere.

Unlike the powder or the green body (compact), the calcined body of this embodiment is composed of fused particles. Fused particles have a structure indicative of the initial stage of sintering, and the calcined body has a structure in which necks are formed between the particles while the shape of the powder particles contained in the zirconia compositional substance of this embodiment is partly maintained. As a result, the calcined body acquires mechanical properties suitable for mechanical processing.

The shape of the calcined body of this embodiment may be any desired shape suitable for the purpose, for example. The shape of the calcined body is, for example, at least one selected from the group consisting of a disk shape, a columnar shape, a cubic shape, a cuboid shape, a polyhedral shape, a spherical shape and a substantially spherical shape or a shape of a dental prosthesis.

The measured density (calcined body density) of the calcined body of this embodiment is, for example, 2.8 g/cm$^3$ or more or 3.2 g/cm$^3$ or more and 3.5 g/cm$^3$ or less or 3.4 g/cm$^3$ or less, and is preferably 2.8 g/cm$^3$ or more and 3.5 g/cm$^3$ or less or 3.2 gcm$^3$ or more and 3.4 g/cm$^3$ or less. It should be noted that since densification progresses little in the calcined body, the calcined body density may be about the same as that of the measured density (green body density) of the green body.

The calcined body of this embodiment may have a hardness suitable for CAD/CAM mechanical processing, and, for example, the Vickers hardness thereof is 25 HV or more and 150 HV or less (25 kgf/mm$^2$ or more and 150 kgf/mm$^2$ or less). The Vickers hardness of the calcined body of this embodiment is preferably 30 HV or more, 40 HV or more or 45 HV or more and 70 HV or less, 60 HV or less, 55 HV or less or 50 HV or less.

The variation in the Vickers hardness between the calcined bodies prepared under the same conditions from the zirconia compositional substance of this embodiment having the same composition is preferably small, and the standard deviation of the Vickers hardness among the calcined bodies is preferably 4 HV or less and 3 HV or less. The standard deviation of the Vickers hardness is preferably small, and is, for example, 0 HV or more or more than 0 HV.

In addition, the difference in hardness among the calcined bodies of this embodiment caused by the compositional difference is preferably small; for example, the absolute value (hereinafter may also be referred to as the "hardness difference") of the difference between the hardness of a calcined body obtained from a commercially available powder (trade name: Zpex 4 produced by Tosoh Corporation) under the following forming conditions and calcining conditions and the hardness of a calcined body obtained from the zirconia compositional substance of this embodiment under the same forming conditions and calcining conditions is preferably 10 HV or less, 9 HV or less, 7 HV or less, 6 HV or less or 5 HV or less. The hardness difference is preferably as small as possible, and is, for example, 0 HV or more, more than 0 HV, 1 HV or more or 2 HV or more. The hardness difference between the calcined bodies of this embodiment is, for example, 0 HV or more and 10 HV or less, more than 0 HV and 9 HV or less or 2 HV or more and 5 HV or less.
(Forming conditions) forming method: uniaxial pressing and CIP process
Uniaxial pressing pressure: 49 $\pm$ 3 MPa
CIP pressure: 196 $\pm$ 5 MPa
(Calcining conditions) calcining method: pressureless firing
Atmosphere: air atmosphere
Firing temperature: 1000°C
Firing time: 1 hour
Heating rate: 50 $\pm$ 5°C/hour
Cooling rate: 300 $\pm$ 10°C/hour

The calcined body of this embodiment can be used in known applications, for example, as a calcined body for a dental prosthesis or as a dental mill blank.

[Sintered body and production method therefor]

Either or both of the zirconia compositional substance and the calcined body of this embodiment can be used as a precursor of a sintered body. Either or both of the zirconia compositional substance and the calcined body of this embodiment can be used in a method for producing a sintered body, the method including a step of sintering either or both of the zirconia compositional substance and the calcined body of this embodiment (hereinafter, this step may also be referred to as the "sintering step").
A sintered body (hereinafter may also be referred to as the "sintered body of this embodiment") is obtained by calcining either or both of the zirconia compositional substance and the calcined body.
Any desired sintering method by which densification of zirconia progresses can be used as the sintering method in the sintering step. The sintering method is preferably at least one sintering method selected from the group consisting of pressure sintering, vacuum sintering and pressureless sintering, is more preferably pressureless sintering and yet more preferably pressureless sintering in an air atmosphere. A sintered body can be obtained as a pressureless sintered body by pressureless sintering.
Preferable conditions for the pressureless sintering are as follows.
Sintering atmosphere: atmosphere other than reducing atmosphere, preferably oxidizing atmosphere and more preferably air atmosphere

Process temperature: higher than 1200°C, 1300°C or higher or 1400°C or higher and
1600°C or lower, 1550°C or lower or 1500°C or lower
Heating rate: 50°C/hour or more, 100°C/hour or more or 150°C/hour or more and
800°C/hour or less or 700°C/hour or less

The process temperature retention time (hereinafter may also be referred to as the "sintering time") may be appropriately adjusted according to the shape, size and amount of the zirconia compositional substance subjected to sintering and the type and performance of a sintering furnace. The sintering time is, for example, 0.5 hours or more or 1 hour or more and 5 hours or less or 3 hours or less.
Other preferable sintering conditions are as follows.
Sintering atmosphere: atmosphere other than reducing atmosphere, preferably oxidizing atmosphere and more preferably air atmosphere

Process temperature: higher than 1200°C, 1300°C or higher or 1400°C or higher and
1600°C or lower, 1550°C or lower or 1500°C or lower
Heating rate: 30°C/minute or more or 50°C/minute or more and
300°C/minute or less 250°C/minute or less
Sintering time: 1 minute or more or 5 minutes or more and
1 hour or less or 0.5 hours or less

The sintered body of this embodiment may have the same composition as the zirconia compositional substance and the calcined body described above.
The sintered body of this embodiment preferably has a color tone similar to any color tone in the dental shade guide. The sintered body of this embodiment more preferably has a color tone of A1, A2, A3, A3.5, A4, B1, B2, B3, B4, C1, C2, C3, C4, D2, D3 or D4 in a dental shade guide (for example, VITA Classical shade guide).

The color tone of the sintered body differs depending on its light transmitting property. For example, when the color tones of the dental shade guide for the sintered bodies having a total transmittance of 24% to 44% are indicated according to the L*a*b* color system, the following color tones can be described as examples.

[Table 1]

| Color tone | L* | a | b* | Color tone | L* | a | b* |
|---|---|---|---|---|---|---|---|
| A1 | 88.0-92.0 | -2.0-1.7 | 12.0-15.4 | C1 | 84.0-87.9 | -2.0-4.0 | 9.0-14.9 |
| A2 | 86.1-90.0 | 1.8-5.0 | 15.0-19.5 | C2 | 80.0-83.9 | 0-7.0 | 15.0-17.4 |
| A3 | 84.5-87.0 | 0-7.0 | 22.5-27.0 | C3 | 77.0-79.8 | 0-6.0 | 17.0-23.0 |
| A3.5 | 82.0-83.9 | 2.0-8.0 | 24.0-26.0 | C4 | 71.0-75.0 | 2.0-6.0 | 17.0-23.0 |
| A4 | 73.0-77.0 | 6.1-10.0 | 19.0-25.0 | D2 | 79.9-81.9 | 0-7.0 | 17.5-22.0 |
| B1 | 89.1-92.0 | -4.0-1.7 | 15.5-21.0 | D3 | 80.0-81.9 | 3.0-9.0 | 22.1-26.0 |
| B2 | 86.1-89.0 | -2.0-1.7 | 18.0-22.4 | D4 | 82.0-83.9 | 0-7.0 | 20.0-23.9 |
| B3 | 84.0-86.0 | 0-7.0 | 16.0-21.9 | | | | |
| B4 | 81.0-84.4 | 1.0-7.0 | 26.1-31.0 | | | | |

The sintered body of this embodiment preferably has a strength suitable for the dental prostheses, and the three-point flexural strength is preferably 550 MPa or more, 600 MPa or more or 800 MPa or more. The three-point flexural strength is, for example, less than 1200 MPa, less than 1100 MPa or 1000 MPa or less and is preferably 550 MPa or more and less than 1200 MPa or 800 MPa or more and 1000 MPa or less.

The sintered body of this embodiment may have a light transmitting property that gives the same aesthetic merit as the aesthetic merit of natural teeth. Such a total transmittance is, for example, 10% or more, 15% or more or 25% or more and 40% or less, 35% or less or 30% or less, and is preferably 10% or more and 40% or less, 15% or more and 35% or less or 25% or more and 30% or less.

Furthermore, the sintered body of this embodiment preferably has a small difference in total transmittance caused by the compositional difference. For example, the ratio (hereinafter may also be referred to as the "transmittance ratio") of the total transmittance of the sintered body obtained from the zirconia compositional substance of this embodiment under the following forming conditions, calcining conditions and sintering conditions to the total transmittance of a sintered body obtained from a commercially available powder (trade name: Zpex 4 produced by Tosoh Corporation) under the same forming conditions, calcining conditions and sintering conditions is 0.5 or more, 0.6 or more or 0.7 or more. The transmittance ratio is preferably as small as possible, but a sintered body with a dark color tone tends to have a large transmittance ratio. Thus, the transmittance ratio is, for example, 1.0 or less of 0.9 or less and is preferably 0.5 or more and 1.0 or less or 0.7 or more and 0.9 or less.

(Forming conditions) forming method: uniaxial pressing and CIP process

Uniaxial pressing pressure: 49 ± 3 MPa
CIP pressure: 196 ± 5 MPa
(Calcining conditions) calcining method: pressureless firing
Atmosphere: air atmosphere
Firing temperature: 1000°C
Firing time: 1 hour
Heating rate: 50 ± 5°C/hour
Cooling rate: 300 ± 10°C/hour
(Sintering conditions) sintering method: pressureless sintering
Atmosphere: air atmosphere
Process temperature: 1450°C
Sintering time: 2 hours
Heating rate: 600 ± 20°C/hour
Cooling rate: 600 ± 50°C/hour

The sintered body of this embodiment can be applied to known usage of zirconia, for example, structural materials, optical materials, dental materials, decorative materials and outer packaging members of electronic appliances, and is preferably used as a dental material and more preferably as a dental prosthesis.

Although embodiments of the present disclosure are described heretofore, the present disclosure is not limited by these embodiments in any way. For example, embodiments in which the constitutional requirements specifically disclosed in the aforementioned embodiments are combined as desired and numerical ranges with upper limits and lower limits combined as desired are also included in the present disclosure. In addition, aspects in which the upper limit and/or the lower limit is replaced by values of the examples described below are also included in the present disclosure.

EXAMPLES

Hereinafter, the embodiment is described in detail through examples. However, this embodiment is not limited to these examples.

(Compositional analysis)

The composition of the compositional substance was measured by ICP analysis. As a pretreatment of the analysis, a sample powder was heat-treated in an air atmosphere at 1000°C for 1 hour.

(BET specific surface area)

The BET specific surface area was determined by a BET multipoint method (5 points) by using an automatic specific surface area automatic analyzer (apparatus name: TriStar II 3020 produced by Shimadzu Corporation) in accordance with JIS R 1626 under the following conditions:

Adsorption medium: $N_2$
Adsorption temperature: -196°C
Pretreatment conditions: air atmosphere, degassing treatment at 250°C for 1 hour or longer

(Crystal phases and tetragonal + cubic phase ratio)

The crystal phases were identified by using an X-ray diffractometer (apparatus name: Ultima IV produced by RIGAKU Corporation) by XRD measurement under the following conditions.
Radiation source: CuK$\alpha$ radiation ($\lambda$ = 0.15418 nm)
Measurement mode: continuous scan
Scan speed: 2°/minute
Measurement range:

2$\theta$ = 26° to 33°
28 = 72° to 76°

Accelerating voltage/current: 40 mA/40 kV
Divergence height slit: 10 mm
Divergence/incident slit: 1°
Receiving slit: open
Detector: semiconductor detector (D/teX Ultra)
Filter: Ni filter
Goniometer radius: 185 mm

Identification of the crystal phases was done by smoothing and background subtraction using an analysis program (program name: Integrated Analytical Software for Powder X-ray Diffraction, PDXL Ver. 2.2 produced by RIGAKU Corporation) that came with the X-ray diffractometer and then profile-fitting the processed XRD pattern by using a split pseudo-Voigt function.

The tetragonal + cubic phase ratio (T + C phase ratio) was determined from the equation described above from the XRD pattern taken from the surfaces of the powder compositional substance, the calcined body, and the sintered body of this embodiment.

(Average granule size)

The average granule size was measured by a laser diffraction/scattering particle size distribution measurement by using a microtrac particle size distribution meter (apparatus name: MT3100II produced by MicrotracBEL Corp.). The measurement conditions were as follows.
Light source: semiconductor laser (wavelength: 780 nm)
Voltage: 3 mW
Refractive index of zirconia: 2.17
Calculation mode: MT3000

A granular powder in a slow aggregation state was directly used as the measurement sample without performing dispersing such as an ultrasonic process.

(Measured density)

The masses of the green body and the calcined body were measured with a balance, and the volumes were determined from the dimensions measured with a caliper. The measured densities were determined from the obtained masses and volumes as the green body density for the green body and the calcined body density for the calcined body.

(Vickers hardness)

The Vickers hardness was measured by using a Vickers hardness tester (apparatus name: Q30A produced by Qness GmbH) by statically pressing the indenter into a measurement sample surface and measuring the diagonal length of the indentation mark formed in the measurement sample surface under the following conditions. The obtained diagonal length was used to determine the Vickers hardness from the equation described above.
Measurement sample: a disk shape with a thickness of $3.0 \pm 0.5$ mm
Measurement load: 1 kgf
Load retention time: 5 seconds

Measurement was conducted on ten points in each of ten green bodies or calcined bodies prepared under the same conditions, and the average thereof was assumed to be the Vickers hardness. Prior to the measurement, the measurement surface of the measurement sample was pretreated by being polished with a #800 water-resistant abrasive paper to remove irregularities exceeding 0.1 mm.

(Total transmittance)

The total transmittance was measured with a haze meter (apparatus name: NDH4000 produced by NIPPON DENSHOKU INDUSTRIES Co., Ltd.) using a D65 light source by a method in conformity with JIS K 7361-1. The measurement sample was a disk-shaped sintered body having both surfaces polished to surface roughness $Ra \leq 0.02 \mu m$ and having a thickness of $1.0 \pm 0.1$ mm.

(Color tone)

The color tone was measured with a spectrophotometric colorimeter (apparatus name: CM-700d produced by KONICA MINOLTA JAPAN, INC.) equipped with an Illumination/Receiving optical system in conformity with the geometrical condition c of JIS Z 8722. The measurement conditions were as follows.
Light source: D65 light source
Viewing angle: 10°
Measurement method: SCI

After a desired site of the sintered body is cut out in a horizontal direction as a measurement sample, both surfaces of this sample was mirror-polished to a diameter of 20 mm × thickness of $1.0 \pm 0.1$ mm and a surface roughness (Ra) of 0.02 μm or less. The measurement sample was placed on a white board, both polished surfaces were used as the evaluation surfaces, and the color tone (L*, a* and b*) was measured from each surface (white background measurement). The color evaluation effective area employed was a diameter of 10 mm.

(Three-point flexural strength)

The three-point flexural strength was measured by a method in accordance with JIS R 1601. The measurement sample had a columnar shape 4 mm in width, 3 mm in thickness and 45 mm in length. Measurement was carried out by applying a load in a horizontal direction of the measurement sample with a support span of 30 mm.

<Synthesis of raw material powders>

Synthesis example 1

Powders 1 to 9 were synthesized by the following method. That is, a hydrated zirconia aqueous solution obtained by hydrolysis of a zirconium oxychloride aqueous solution, an iron(III) chloride aqueous solution having an $FeCl_3$ concentration of 45 mass% and yttrium chloride were mixed to compositions of the powders indicated in the table below so as to obtain raw material solutions. Each of the raw material solutions was dried in an air circulating atmosphere at 180°C to prepare dry powders, and the dry powders were heat-treated at temperatures indicated in the table below in the air atmosphere to obtain calcined powders. Pure water and 199.9 g of the obtained calcined powder were mixed in a ball mill, and, thereto, an acrylic resin and, if necessary, either or both of a slurry containing an α-alumina powder and a slurry containing titanium oxide powder were added to prepare each slurry. The slurry was spray-dried in an air atmosphere at 180°C to obtain nine types of granular powders (powders 1 to 9).

Synthesis example 2

A raw material solution having a composition of the powder indicated in the table below was obtained as in synthesis example 1 except that the iron(III) chloride aqueous solution was not used, and dried in an air circulating atmosphere at 180°C to prepare a dry powder, and the dry powder was heat-treated at a temperature of 1175°C in the air atmosphere to obtain a calcined powder.

199.9 g of the obtained calcined powder, 0.1 g of an α-alumina powder, 0.45 g of iron oxyhydroxide (FeOOH) powder and pure water were mixed in a ball mill, and then an acrylic resin was added thereto to prepare a slurry. The slurry was spray-dried in an air atmosphere at 180°C to obtain a granular powder (powder 10).

The results of synthesis examples 1 and 2 are indicated in the table below.

[Table 2]

| | Calcining temperature [°C] | Iron source | $Y_2O_3$ [mol%] | $Fe_2O_3$ [wt%] | $TiO_2$ [wt%] | $Al_2O_3$ [wt%] | Average particle size [μm] | Granular powder | | T+C phase ratio [%] |
|---|---|---|---|---|---|---|---|---|---|---|
| | | | | | | | | Average granule size [μm] | BET specific surface area [m²/g] | |
| Powder 1 | 1125 | $FeCl_3$ | 4.0 | 0.20 | - | 0.05 | 0.45 | 44 | 10.5 | 94 |
| Powder 2 | 1125 | $FeCl_3$ | 4.0 | 0.20 | 0.95 | 0.05 | 0.45 | 44 | 10.5 | 94 |
| Powder 3 | 1135 | $FeCl_3$ | 3.0 | 0.15 | - | 0.05 | 0.44 | 44 | 10.5 | 70 |
| Powder 4 | 1110 | $FeCl_3$ | 5.2 | 0.25 | - | 0.05 | 0.43 | 43 | 10.6 | 95 |
| Powder 5 | 1130 | $FeCl_3$ | 5.2 | 0.18 | - | - | 0.43 | 44 | 10.6 | 95 |
| Powder 6 | 1120 | $FeCl_3$ | 5.2 | 0.21 | - | - | 0.46 | 44 | 10.8 | 95 |

(continued)

| | Calcining temperature [°C] | Iron source | $Y_2O_3$ [mol%] | $Fe_2O_3$ [wt%] | $TiO_2$ [wt%] | $Al_2O_3$ [wt%] | Average particle size [μm] | Granular powder | | T+C phase ratio [%] |
|---|---|---|---|---|---|---|---|---|---|---|
| | | | | | | | | Average granule size [μm] | BET specific surface area [m²/g] | |
| Powder 7 | 1110 | $FeCl_3$ | 5.2 | 0.25 | - | - | 0.45 | 43 | 10.9 | 95 |
| Powder 8 | 1095 | $FeCl_3$ | 5.2 | 0.25 | - | - | 0.43 | 45 | 12.5 | 95 |
| Powder 9 | 1110 | $FeCl_3$ | 5.2 | 0.25 | 0.11 | - | 0.46 | 43 | 10.6 | 95 |
| Powder 10 | 1175 | FeOOH | 4.0 | 0.20 | - | 0.05 | 0.43 | 44 | 10.2 | 94 |

The powders 1 and 10 both contained iron, and the powder 1 had a high-intensity percentage of 0.05%, a coarse intensity percentage of 0%, a maximum intensity ratio of 0.107 and a minimum X-ray intensity ratio of 0. In contrast, the powder 10 had a high-intensity percentage of 2.04%, a coarse intensity percentage of 0.54%, a maximum intensity ratio of 0.901 and a minimum intensity ratio of 0.

Synthesis example 3

A raw material solution having a composition of the powder indicated in the table below was obtained as in synthesis example 1 except that a nickel oxide (NiO) powder was used instead of the iron(III) chloride aqueous solution. The resulting raw material solution was dried in an air circulating atmosphere at 180°C to prepare a dry powder, and then the dry powder was heat-treated at a temperature of 1140°C in the air atmosphere to obtain a calcined powder.

199.9 g of the obtained calcined powder, 0.1 g of an α-alumina powder and pure water were mixed in a ball mill, and then an acrylic resin was added thereto to prepare a slurry. The slurry was spray-dried in an air atmosphere at 180°C to obtain a granular powder (powder 11).

Synthesis example 4

A raw material solution having a composition of the powder indicated in the table below was obtained as in synthesis example 1 except that the nickel oxide (NiO powder) was not used, and dried in an air circulating atmosphere at 180°C to prepare a dry powder, and the dry powder was heat-treated at a temperature of 1140°C in the air atmosphere to obtain a calcined powder.

199.9 g of the obtained calcined powder, 0.1 g of an α-alumina powder, 0.1 g of a nickel oxide powder and pure water were mixed in a ball mill, and then an acrylic resin was added thereto to prepare a slurry. The slurry was spray-dried in an air atmosphere at 180°C to obtain a granular powder (powder 12).

The results of synthesis examples 3 and 4 are indicated in the table below.

[Table 3]

| | Calcining temperature [°C] | Nickel source | $Y_2O_3$ [mol%] | NiO [wt%] | $Al_2O_3$ [wt%] | Average particle size [μm] | Granular powder | | T+C phase ratio [%] |
|---|---|---|---|---|---|---|---|---|---|
| | | | | | | | Average granule size [μm] | BET specific surface area [m²/g] | |
| Powder 11 | 1140 | NiO | 4.0 | 0.05 | 0.05 | 0.44 | 45 | 10.6 | 94 |
| Powder 12 | 1140 | NiO | 4.0 | 0.05 | 0.05 | 0.46 | 45 | 10.1 | 94 |

Synthesis example 5

A raw material solution having a composition of the powder indicated in the table below was obtained as in synthesis example 1 except that a tricobalt tetraoxide ($Co_3O_4$) powder was used instead of the iron(III) chloride aqueous solution, and dried in an air circulating atmosphere at 180°C to prepare a dry powder, and the dry powder was heat-treated at a temperature of 1140°C in the air atmosphere to obtain a calcined powder.

199.9 g of the obtained calcined powder, 0.1 g of an $\alpha$-alumina powder and pure water were mixed in a ball mill, and then an acrylic resin was added thereto to prepare a slurry. The slurry was spray-dried in an air atmosphere at 180°C to obtain a granular powder (powder 13).

Synthesis example 6

Raw material solutions having compositions of the powders indicated in the table below were obtained as in synthesis example 1 except that the iron(III) chloride aqueous solution was not used, and dried in an air circulating atmosphere at 180°C to prepare dry powders, and the dry powders were heat-treated at calcining temperatures indicated in the table below in the air atmosphere to obtain calcined powders.

199.9 g of the calcined powder, 0.1 g of an $\alpha$-alumina powder, 0.1 g of a cobalt oxide powder and pure water were mixed in a ball mill, and then an acrylic resin was added thereto to prepare a slurry. The slurry was spray-dried in an air atmosphere at 180°C to obtain granular powders (powders 14 and 15).

Furthermore, a granular powder (powder 16) was obtained by in the same manner except that 199.9 g of the calcined powder, 0.1 g of an $\alpha$-alumina powder, 0.08 g of a cobalt oxide powder, 0.4 g of a titanium oxide powder and pure water were mixed in a ball mill.

The results of synthesis examples 5 and 6 are indicated in the table below.

[Table 4]

| | Calcining temperature [°C] | Cobalt source | $Y_2O_3$ [mol%] | $Co_3O_4$ [wt%] | $TiO_2$ [wt%] | $Al_2O_3$ [wt%] | Average particle size [μm] | Granular powder | | T+C phase ratio [%] |
|---|---|---|---|---|---|---|---|---|---|---|
| | | | | | | | | Average granule size [μm] | BET specific surface area [m²/g] | |
| Powder 13 | 1140 | $Co_3O_4$ | 4.0 | 0.05 | - | 0.05 | 0.44 | 45 | 10.3 | 94 |
| Powder 14 | 1105 | $Co_3O_4$ | 3.0 | 0.04 | - | 0.05 | 0.44 | 44 | 13.0 | 70 |
| Powder 15 | 1175 | $Co_3O_4$ | 4.0 | 0.04 | 0.2 | 0.05 | 0.45 | 44 | 10.0 | 94 |
| Powder 16 | 1175 | $Co_3O_4$ | 4.0 | 0.04 | - | 0.05 | 0.44 | 44 | 10.0 | 94 |

Synthesis example 7

A raw material solution having a composition of the powder indicated in the table below was obtained as in synthesis example 1 except that a trimanganese tetraoxide ($Mn_3O_4$) powder was used instead of the iron(III) chloride aqueous solution, and dried in an air circulating atmosphere at 180°C to prepare a dry powder, and the dry powder was heat-treated at a temperature of 1140°C in the air atmosphere to obtain a calcined powder.

199.9 g of the obtained calcined powder, 0.1 g of an $\alpha$-alumina powder and pure water were mixed in a ball mill, and then an acrylic resin was added thereto to prepare a slurry. The slurry was spray-dried in an air atmosphere at 180°C to obtain a granular powder (powder 17).

Synthesis example 8

A raw material solution having a composition of the powder indicated in the table below was obtained as in synthesis example 1 except that the trimanganese tetraoxide powder was not used, and dried in an air circulating atmosphere at 180°C to prepare a dry powder, and the dry powder was heat-treated at a temperature of 1175°C in the air atmosphere to obtain a calcined powder.

199.9 g of the obtained calcined powder, 0.1 g of an α-alumina powder, 0.1 g of a trimanganese tetraoxide powder and pure water were mixed in a ball mill, and then an acrylic resin was added thereto to prepare a slurry. The slurry was spray-dried in an air atmosphere at 180°C to obtain a granular powder (powder 18).

The results of synthesis examples 7 and 8 are indicated in the table below.

[Table 5]

| | Calcining temperature [°C] | Manganese source | $Y_2O_3$ [mol%] | $Mn_3O_4$ [wt%] | $Al_2O_3$ [wt%] | Average particle size [μm] | Granular powder | | T+C phase ratio [%] |
| | | | | | | | Average granule size [μm] | BET specific surface area [m$^2$/g] | |
|---|---|---|---|---|---|---|---|---|---|
| Powder 17 | 1140 | $Mn_3O_4$ | 4.0 | 0.05 | 0.05 | 0.44 | 45 | 10.5 | 94 |
| Powder 18 | 1140 | $Mn_3O_4$ | 4.0 | 0.05 | 0.05 | 0.44 | 45 | 10.0 | 94 |

Synthesis example 9

A raw material solution having a composition of the powder indicated in the table below was obtained as in synthesis example 1 except that the iron(III) chloride aqueous solution was not used and that erbium oxide was used instead of yttrium oxide, and dried in an air circulating atmosphere at 180°C to prepare a dry powder, and the dry powder was heat-treated at a calcining temperature indicated in the table below in the air atmosphere to obtain a calcined powder.

199.9 g of the obtained calcined powder, 0.1 g of an α-alumina powder and pure water were mixed in a ball mill, and then an acrylic resin was added thereto to prepare a slurry. The slurry was spray-dried in an air atmosphere at 180°C to obtain granular powders (powders 19 and 20). The results are indicated in the table below.

[Table 6]

| | Calcining temperature [°C] | $Er_2O_3$ [wt%] | $Al_2O_3$ [wt%] | Average particle size [μm] | Granular powder | | T+C phase ratio [%] |
| | | | | | Average granule size [μm] | BET specific surface area [m$^2$/g] | |
|---|---|---|---|---|---|---|---|
| Powder 19 | 1105 | 9.3 (3.2mol%) | 0.05 | 0.45 | 43 | 12.1 | 60 |
| Powder 20 | 1175 | 11.7 (4.1mol%) | 0.05 | 0.46 | 42 | 9.8 | 92 |

Synthesis example 10

Raw material solutions having compositions of the powders indicated in the table below were obtained as in synthesis example 1 except that the iron(III) chloride aqueous solution was not used, and dried in an air circulating atmosphere at 180°C to prepare dry powders, and the dry powders were heat-treated at calcining temperatures indicated in the table below in the air atmosphere to obtain calcined powders.

199.9 g of the obtained calcined powder, 0.1 g of an α-alumina powder and pure water were mixed in a ball mill, and then an acrylic resin was added thereto to prepare a slurry. The slurry was spray-dried in an air atmosphere at 180°C to obtain granular powders (powders 21 and 23).

In addition, a commercially available zirconia powder (product name: Zpex4 produced by TOSOH CORPORATION) was used as a powder 22. The compositions and physical properties of the granular powders are indicated in the table below.

[Table 7]

|  | Calcining temperature [°C] | $Y_2O_3$ [mol%] | $Al_2O_3$ [wt%] | Average particle size [μm] | Granular powder | | T+C phase ratio [%] |
|---|---|---|---|---|---|---|---|
|  |  |  |  |  | Average granule size [μm] | BET specific surface area [m²/g] |  |
| Powder 21 | 1175 | 3.0 | 0.05 | 0.47 | 44 | 10.0 | 70 |
| Powder 22 |  | 4.0 | 0.05 | 0.46 | 44 | 10.0 | 75 |
| Powder 23 | 1175 | 5.2 | 0.05 | 0.46 | 44 | 10.0 | 95 |

Examples 1 to 7 and Comparative Examples 1 to 3

Powders in mass percentages indicated in the table below were charged in a 200 mL polypropylene container and dry-mixed by stirring the container so as to obtain powder compositional substances of examples and comparative examples. The results are indicated in the table below.

[Table 8]

|  | Powder compositional substance [wt%] | | | | Average particle size [μm] | BET specific surface area [m²/g] | Average granule size [μm] | Bulk density [g/cm³] | T+C phase ratio [%] |
|---|---|---|---|---|---|---|---|---|---|
| Example 1 | Powder 1 | Powder 14 | Powder 19 | Powder 22 | 0.43 | 10.3 | 44 | 1.27 | 79 |
|  | 23.50 | 6.60 | 1.08 | 68.82 |  |  |  |  |  |
| Example 2 | Powder 1 | Powder 14 |  | Powder 22 | 0.46 | 10.6 | 44 | 1.27 | 83 |
|  | 47.00 | 11.20 |  | 41.80 |  |  |  |  |  |
| Example 3 | Powder 1 | Powder 14 | Powder 19 | Powder 22 | 0.45 | 10.8 | 44 | 1.28 | 86 |
|  | 64.00 | 15.27 | 1.08 | 19.65 |  |  |  |  |  |
| Example 4 | Powder 2 | Powder 16 | Powder 20 | Powder 22 | 0.43 | 10.1 | 44 | 1.27 | 81 |
|  | 25.50 | 6.60 | 0.84 | 67.06 |  |  |  |  |  |
| Example 5 | Powder 2 | Powder 16 |  | Powder 22 | 0.43 | 10.3 | 44 | 1.27 | 87 |
|  | 50.00 | 11.20 |  | 38.80 |  |  |  |  |  |
| Example 6 | Powder 2 | Powder 16 | Powder 20 | Powder 22 | 0.44 | 10.3 | 44 | 1.27 | 91 |
|  | 68.00 | 15.17 | 0.84 | 15.99 |  |  |  |  |  |
| Comparative Example 1 | Powder 10 | Powder 14 | Powder 19 | Powder 22 | 0.44 | 10.3 | 44 | 1.27 | 79 |
|  | 23.50 | 6.60 | 1.08 | 68.82 |  |  |  |  |  |
| Comparative Example 2 | Powder 10 | Powder 14 |  | Powder 22 | 0.43 | 10.4 | 44 | 1.27 | 83 |
|  | 47.00 | 11.20 |  | 41.80 |  |  |  |  |  |

(continued)

| | Powder compositional substance [wt%] | | | | Average particle size [$\mu$m] | BET specific surface area [$m^2$/g] | Average granule size [$\mu$m] | Bulk density [g/$cm^3$] | T+C phase ratio [%] |
|---|---|---|---|---|---|---|---|---|---|
| Comparative Example 3 | Powder 10 | Powder 14 | Powder 19 | Powder 22 | 0.42 | 10.3 | 44 | 1.27 | 86 |
| | 64.00 | 15.27 | 1.08 | 19.65 | | | | | |

Examples 1 and 4 and Comparative Example 1 are powders having compositions corresponding to a sintered body having color tone C1 in the dental shade guide. Examples 2 and 5 and Comparative Example 2 are powders having compositions corresponding to a sintered body having color tone C3 in the dental shade guide. Examples 3 and 6 and Comparative Example 3 are powders having compositions corresponding to a sintered body having color tone C4 in the dental shade guide.

The high-intensity percentage was 0% (0.002%) in Example 1, 0.02% in Example 2 and 0.03% in Example 3, and it could be confirmed that the high-intensity percentage increased with the increase in the amount of $Fe_2O_3$. Meanwhile, the high-intensity percentage of Comparative Example 3 was 3.06%, more than 100 times that in Example 3. The coarse intensity percentage was 0% in all of Examples 1 to 3 but was 0.38% in Comparative Example 3; thus it could be confirmed that aggregation of iron was notable in Comparative Example 3 compared to Examples.

The maximum intensity ratio was 0.053 in Example 1, 0.054 in Example 2, 0.057 in Example 3 and 0.334 in Comparative Example 3, and the minimum intensity ratio was 0.001 in Example 1, 0 in Example 2, 0 in Example 3 and 0.002 in Comparative Example 3. As a result, it could be confirmed that, compared to the examples, the comparative example contains iron (M1 element) in various aggregated states since the intensity ratio range was 0.052 for Example 1, 0.054 for Example 2 and 0.057 for Example 3 whereas the intensity ratio range was 0.332 in Comparative Example 3.

3.0 g of each obtained powder was charged in a mold having a diameter of 25 mm, uniaxially press-formed at a pressure of 49 MPa, and then subjected to a CIP process at a pressure of 196 MPa to obtain a disk-shaped green body (compact). These green bodies were calcined under the following conditions to obtain calcined bodies of the examples and the comparative examples.

Calcining temperature: 1000°C

Calcining time: 1 hour

Heating rate: 50°C/hour

Calcining atmosphere: air atmosphere

Cooling rate: 300°C/hour

The calcined bodies of Examples 1 and 4 and Comparative Example 1 are indicated as C1 calcined bodies, calcined bodies of Examples 2 and 5 and Comparative Example 2 are indicated as C3 calcined bodies, and calcined bodies of Examples 3 and 6 and Comparative Example 3 are indicated as C4 calcined bodies. The results are indicated in the table below. Note that in the composition in the table below, the contents (in terms of oxide) of elements other than zirconia are shown.

[Table 9]

| | Composition | | | | | | Green body | | Calcined body density [g/$cm^3$] |
|---|---|---|---|---|---|---|---|---|---|
| | $Y_2O_3$ [mol%] | $Er_2O_3$ [wt%] | $Fe_2O_3$ [wt%] | $Co_3O_4$ [wt%] | $Al_2O_3$ [wt%] | $TiO_2$ [wt%] | Density [g/$cm^3$] | Vickers hardness [HV] | |
| Example 1 | 3.950 | 0.100 (0.033mol%) | 0.047 | 0.003 | 0.05 | 0 | 3.31 | 11.6 | 3.30 |

(continued)

| | Composition | | | | | | Green body | | Calcined body density [g/cm³] |
|---|---|---|---|---|---|---|---|---|---|
| | Y₂O₃ [mol%] | Er₂O₃ [wt%] | Fe₂O₃ [wt%] | Co₃O₄ [wt%] | Al₂O₃ [wt%] | TiO₂ [wt%] | Density [g/cm³] | Vickers hardness [HV] | |
| Example 2 | 3.945 | 0 | 0.094 | 0.004 | 0.05 | 0 | 3.30 | 11.9 | 3.30 |
| Example 3 | 3.860 | 0.100 (0.033mol%) | 0.128 | 0.006 | 0.05 | 0 | 3.29 | 12.6 | 3.30 |
| Example 4 | 4.042 | 0.098 (0.033mol%) | 0.051 | 0.003 | 0.05 | 0.255 | 3.32 | 11.6 | 3.28 |
| Example 5 | 4.087 | 0 | 0.100 | 0.004 | 0.05 | 0.497 | 3.31 | 11.8 | 3.27 |
| Example 6 | 4.064 | 0.098 (0.033mol%) | 0.136 | 0.006 | 0.05 | 0.676 | 3.30 | 11.8 | 3.27 |
| Comparative Example 1 | 3.950 | 0.100 (0.033mol%) | 0.047 | 0.003 | 0.05 | 0 | 3.32 | 10.5 | 3.34 |
| Comparative Example 2 | 3.945 | 0 | 0.094 | 0.004 | 0.05 | 0 | 3.32 | 10.5 | 3.37 |
| Comparative Example 3 | 3.860 | 0.100 (0.033mol%) | 0.128 | 0.006 | 0.05 | 0 | 3.32 | 10.6 | 3.41 |

Although the green bodies of the examples have about the same densities as those of the comparative examples, the green bodies of the examples had Vickers hardness as high as 11.5 HV or more, and it could be confirmed that defects rarely occur in these green bodies.

Measurement example 1

The Vickers hardness of the calcined bodies of the examples and the comparative examples was measured. The results are indicated in the table below. The Vickers hardness was measured on ten calcined bodies obtained by repeating the procedures of the examples and the comparative examples. Furthermore, a calcined body was prepared as in the examples except that the powder 10 was used, and the difference from the Vickers hardness (46.3 HV) of the obtained calcined body (hereinafter may also be referred to as the "reference calcined body") is indicated as the hardness difference.

[Table 10]

| | | Vickers hardness [HV] | | Hardness difference [HV] |
|---|---|---|---|---|
| | | Average particle size value | Standard deviation | |
| C1 calcined body | Example 1 | 47.3 | 2.2 | 1.0 |
| | Example 4 | 47.6 | 2.1 | 1.3 |
| | Comparative Example 1 | 53.4 | 3.0 | 7.1 |
| C3 calcined body | Example 2 | 49.5 | 2.7 | 3.2 |
| | Example 5 | 49.2 | 2.8 | 2.9 |
| | Comparative Example 2 | 62.0 | 3.3 | 15.7 |
| C4 calcined body | Example 3 | 50.8 | 3.1 | 4.5 |
| | Example 6 | 50.6 | 3.0 | 4.3 |
| | Comparative Example 3 | 65.8 | 5.1 | 19.5 |

It can be confirmed that, according to the calcined bodies of the examples and the comparative examples, the Vickers hardness increases and the standard deviation tends to increase from the C1 calcined bodies to the C4 calcined bodies as the color tone of the calcined body darkens. Moreover, in any color tone, the calcined bodies of the examples have lower Vickers hardness and a smaller standard deviation than the calcined bodies of the comparative examples, and thus it could be confirmed that variation in hardness between the production lots was reduced.

Moreover, the Vickers hardness of the calcined bodies of the examples for the C1 to C4 calcined bodies was 47.3 HV to 50.8 HV with a hardness difference being 5 HV or less whereas the Vickers hardness of the calcined bodies of the comparative examples was 53.4 HV to 65.8 HV with a hardness difference exceeding 10 HV.

The hardness difference from the calcined body obtained from the commercially available zirconia powder was 1.0 HV to 4.5 HV, that is, 5 HV or less, in the examples. In contrast, in the comparative examples, the hardness difference was as large as 7.1 HV to 19.5 HV, that is, 7 HV or more, and it can be confirmed that this difference widens as the color tone darkens.

<Preparation of sintered body>

The calcined bodies of the examples and the comparative examples and the reference calcined body were sintered under the following conditions to obtain sintered bodies.

Sintering method: pressureless sintering
Sintering temperature: 1500°C
Sintering time: 2 hours
Heating rate: 600°C/hour
Sintering atmosphere: air atmosphere

Of the obtained sintered bodies, the sintered bodies of Examples 1 and 4 and Comparative Example 1 are indicated as C1 sintered bodies, sintered bodies of Examples 2 and 5 and Comparative Example 2 are indicated as C3 sintered bodies, and sintered bodies of Examples 3 and 6 and Comparative Example 3 are indicated as C4 sintered bodies. A sintered body obtained from the reference calcined body was indicated as a reference sintered body. The results are indicated in the table below. In the table below, the transmittance ratio represents the total transmittance of each sintered body with respect to the total transmittance (42%) of the reference sintered body.

[Table 11]

| | | Color tone | | | Three-point flexural strength (MPa) | Transmittance ratio |
|---|---|---|---|---|---|---|
| | | L* | a | b* | | |
| C1 sintered body | Example 1 | 86.0 | 1.3 | 11.4 | 1050 | 0.86 |
| | Example 4 | 86.7 | 1.2 | 11.0 | 1060 | 0.86 |
| | Comparative Example 1 | 86.1 | 1.4 | 12.0 | 1060 | 0.86 |
| C3 sintered body | Example 2 | 78.9 | 2.6 | 18.7 | 1070 | 0.71 |
| | Example 5 | 79.7 | 2.4 | 18.2 | 1060 | 0.71 |
| | Comparative Example 2 | 79.1 | 2.7 | 18.9 | 1070 | 0.71 |
| C4 sintered body | Example 3 | 73.0 | 4.9 | 20.9 | 1070 | 0.62 |
| | Example 6 | 73.9 | 4.6 | 20.3 | 1070 | 0.62 |
| | Comparative Example 3 | 73.1 | 4.9 | 21.5 | 1080 | 0.62 |

The sintered bodies of the examples and the comparative examples had about the same transmittance ratio and color tone. As a result, it could be confirmed that a sintered body having a color tone and a total transmittance comparable to those of existing sintered bodies can be obtained from a calcined body of the examples.

Examples 7 to 12

Powders in mass percentages indicated in the table below were charged in a 200 mL polypropylene container and dry-mixed by stirring the container so as to obtain powder compositional substances of examples and comparative examples.

The obtained powder compositional substances were formed and calcined as in Example 1 to obtain calcined bodies of Examples 7 to 12. The results of the powder compositional substances are indicated in Table 12, and the results of the green bodies and the calcined bodies are indicated in Table 13.
Note that in the composition in Table 13, the contents of materials other than zirconia are shown.

[Table 12]

| | Powder compositional substance [wt%] | | | | Average particle size [μm] | BET specific surface area [m$^2$/g] | Average granule size [μm] | Bulk density [g/cm$^3$] | T+C phase ratio [%] |
|---|---|---|---|---|---|---|---|---|---|
| Example 7 | Powder 3 | Powder 14 | Powder 19 | Powder 21 | 0.45 | 10.2 | 44 | 1.27 | 70 |
| | 30.00 | 6.67 | 1.08 | 62.25 | | | | | |
| Example 8 | Powder 3 | Powder 14 | | Powder 21 | 0.46 | 10.3 | 44 | 1.27 | 70 |
| | 60.00 | 10.00 | | 30.00 | | | | | |
| Example 9 | Powder 3 | Powder 14 | Powder 19 | Powder 21 | 0.42 | 10.4 | 44 | 1.27 | 70 |
| | 80.00 | 13.33 | 1.08 | 5.59 | | | | | |
| Example 10 | Powder 4 | Powder 16 | Powder 20 | Powder 23 | 0.43 | 10.1 | 44 | 1.27 | 95 |
| | 15.82 | 5.49 | 0.86 | 77.83 | | | | | |
| Example 11 | Powder 4 | Powder 16 | | Powder 23 | 0.43 | 10.2 | 44 | 1.28 | 95 |
| | 32.45 | 10.99 | | 56.56 | | | | | |
| Example 12 | Powder 4 | Powder 16 | | Powder 23 | 0.43 | 10.3 | 44 | 1.28 | 95 |
| | 43.61 | 15.68 | | 40.71 | | | | | |

The high-intensity percentage was 0.01% (0.005%) in Example 7, 0.01% (0.011%) in Example 8, 0.01% (0.014%) in Example 9, 0.03% in Example 10 and 0.06% in Example 11. This confirmed that there is a tendency for the high-intensity percentage to increase with the increase in the amount of $Fe_2O_3$. Furthermore, the coarse intensity percentage was 0% in all of Examples 7 to 11.

The maximum intensity ratio was 0.012 in Example 7, 0.015 in Example 8, 0.015 in Example 9, 0.057 in Example 10 and 0.128 in Example 11. The minimum intensity ratio was 0 in all Examples 7 to 11, and the intensity ratio range was 0.012 in Example 7, 0.015 in Example 8 and 0.015 in Example 9. There was a tendency of the high-intensity percentage to increase and the intensity ratio range to widen as the stabilizing element amount increased.

[Table 13]

| | Composition | | | | | | Green body | | Calcined body density [g/cm$^3$] |
|---|---|---|---|---|---|---|---|---|---|
| | $Y_2O_3$ [mol%] | $Er_2O_3$ [wt%] | $Fe_2O_3$ [wt%] | $Co_3O_4$ [wt%] | $Al_2O_3$ [wt%] | $TiO_2$ [wt%] | Density [g/cm$^3$] | Vickers hardness [HV] | |
| Example 7 | 2.963 | 0.100 (0.033mol%) | 0.045 | 0.003 | 0.05 | 0.000 | 3.31 | 11.8 | 3.30 |
| Example 8 | 2.995 | 0 | 0.090 | 0.004 | 0.05 | 0.000 | 3.30 | 11.9 | 3.31 |
| Example 9 | 2.965 | 0.100 (0.033mol%) | 0.120 | 0.005 | 0.05 | 0.000 | 3.29 | 11.7 | 3.31 |

(continued)

| | Composition | | | | | | Green body | | Calcined body density [g/cm$^3$] |
| | Y$_2$O$_3$ [mol%] | Er$_2$O$_3$ [wt%] | Fe$_2$O$_3$ [wt%] | Co$_3$O$_4$ [wt%] | Al$_2$O$_3$ [wt%] | TiO$_2$ [wt%] | Density [g/cm$^3$] | Vickers hardness [HV] | |
|---|---|---|---|---|---|---|---|---|---|
| Example 10 | 4.969 | 0.101 (0.034mol%) | 0.040 | 0.002 | 0.05 | 0.011 | 3.31 | 11.5 | 3.27 |
| Example 11 | 4.959 | 0 | 0.081 | 0.004 | 0.05 | 0.022 | 3.30 | 11.1 | 3.28 |
| Example 12 | 4.913 | 0 | 0.109 | 0.006 | 0.05 | 0.031 | 3.30 | 11.2 | 3.28 |

Measurement example 2

Measurement of the Vickers hardness of the calcined bodies and preparation and evaluation of the sintered bodies were performed as in Measurement example 1. The measurement results of the Vickers hardness are indicated in Table 14, and the evaluation results of the sintered bodies are indicated in Table 15.

[Table 14]

| | | Vickers hardness [HV] | | Hardness difference [HV] |
| | | Average particle size value | Standard deviation | |
|---|---|---|---|---|
| C1 calcined body | Example 7 | 47.6 | 1.9 | 1.3 |
| | Example 10 | 47.8 | 2.1 | 1.5 |
| C3 calcined body | Example 8 | 49.6 | 2.8 | 3.3 |
| | Example 11 | 49.9 | 2.8 | 3.6 |
| C4 calcined body | Example 9 | 50.8 | 3.1 | 4.5 |
| | Example 12 | 50.7 | 3.3 | 4.4 |

The calcined bodies of Examples 7 to 12 all exhibited Vickers hardness about the same as those of the calcined bodies of Examples 1 to 6, and the influence of the stabilizing element (yttrium) content on the Vickers hardness could not be confirmed.

[Table 15]

| | | Color tone | | | Three-point flexural strength (MPa) | Transmittance ratio |
| | | L* | a | b* | | |
|---|---|---|---|---|---|---|
| C1 sintered body | Example 7 | 85.9 | 1.2 | 10.6 | 1144 | 0.74 |
| | Example 10 | 83.7 | -1.5 | 11.9 | 820 | 1.00 |
| C3 sintered body | Example 8 | 79.0 | 2.6 | 17.7 | 1153 | 0.57 |
| | Example 11 | 77.0 | -0.2 | 19.3 | 831 | 0.88 |
| C4 sintered body | Example 9 | 73.3 | 5.1 | 18.7 | 1110 | 0.45 |
| | Example 12 | 73.8 | 3.0 | 19.8 | 839 | 0.76 |

It could be confirmed that the increase in the stabilizing element (yttrium) content decreased the three-point flexural strength and increased the transmittance.

Examples 13 and 14

Powders in mass percentages indicated in the table below were charged in a 200 mL polypropylene container and dry-mixed by stirring the container so as to obtain powder compositional substances of examples and comparative examples.

The obtained powder compositional substances were formed and calcined as in Example 1 to obtain calcined bodies of Examples 13 and 14. The results of the powder compositional substances are indicated in Table 16, and the results of the green bodies and the calcined bodies are indicated in Table 17. Note that in the composition in Table 17, the contents of materials other than zirconia are shown.

[Table 16]

| | Powder compositional substance [wt%] | | | Average particle size [μm] | BET specific surface area [m$^2$/g] | Average granule size [μm] | Bulk density [g/cm$^3$] | T+C phase ratio [%] |
|---|---|---|---|---|---|---|---|---|
| Example 13 | Powder 5 | Powder 16 | Powder 23 | 0.45 | 10.1 | 44 | 1.28 | 95 |
| | 45.00 | 10.99 | 44.01 | | | | | |
| Example 14 | Powder 6 | Powder 16 | Powder 23 | 0.45 | 10.2 | 44 | 1.28 | 95 |
| | 38.57 | 10.99 | 50.44 | | | | | |

[Table 17]

| | Composition | | | | | Green body | | Calcined body density [g/cm$^3$] |
|---|---|---|---|---|---|---|---|---|
| | Y$_2$O$_3$ [mol%] | Fe$_2$O$_3$ [wt%] | Co$_3$O$_4$ [wt%] | Al$_2$O$_3$ [wt%] | TiO$_2$ [wt%] | Density [g/cm$^3$] | Vickers hardness [HV] | |
| Example 13 | 4.912 | 0.109 | 0.006 | 0.02 | 0.031 | 1.28 | 11.5 | 3.27 |
| Example 14 | 4.912 | 0.109 | 0.006 | 0.02 | 0.031 | 1.28 | 11.4 | 3.27 |

The powders 5 and 6 have an iron content different from each other, and this could confirm that powder compositional substances and green bodies having various physical properties can be obtained by changing the mixing ratio.

Measurement example 3

Measurement of the Vickers hardness of the calcined bodies and preparation and evaluation of the sintered bodies were performed as in Measurement example 1. The measurement results of the Vickers hardness are indicated in Table 18, and the evaluation results of the sintered bodies are indicated in Table 19.

[Table 18]

| | | Vickers hardness [HV] | | Hardness difference [HV] |
|---|---|---|---|---|
| | | Average particle size value | Standard deviation | |
| C4 calcined body | Example 13 | 50.6 | 3.3 | 4.3 |
| | Example 14 | 50.5 | 3.1 | 4.2 |

[Table 19]

| | | Color tone | | | Three-point flexural strength (MPa) | Transmittance ratio |
|---|---|---|---|---|---|---|
| | | L* | a | b* | | |
| C4 sintered body | Example 13 | 72.5 | 2.7 | 19.5 | 849 | 0.76 |
| | Example 14 | 72.9 | 3.1 | 19.5 | 862 | 0.76 |

It could be confirmed from Examples 13 and 14, that the same calcined bodies and sintered bodies can be obtained from powder compositional substances and calcined bodies having different mixing ratios.

Examples 15 to 17

Powders in mass percentages indicated in the table below were charged in a 200 mL polypropylene container and dry-mixed by stirring the container so as to obtain powder compositional substances of examples and comparative examples.

The obtained powder compositional substances were compacted and calcined as in Example 1 to obtain calcined bodies of Examples 15 and 17. The results of the powder compositional substances are indicated in Table 20, and the results of the green bodies and the calcined bodies are indicated in Table 21. Note that in the composition in Table 21, the contents of materials other than zirconia are shown.

[Table 20]

| | Powder compositional substance [wt%] | | | | Average crystal grain size [μm] | BET specific surface area [m²/g] | Average granule size [μm] | Bulk density [g/cm³] | T+C phase ratio [%] |
|---|---|---|---|---|---|---|---|---|---|
| Example 15 | Powder 8 | Powder 16 | Powder 20 | Powder 23 | 0.45 | 10.5 | 44 | 1.27 | 95 |
| | 15.77 | 5.49 | 0.86 | 77.88 | | | | | |
| Example 16 | Powder 8 | Powder 16 | | Powder 23 | 0.46 | 11.1 | 44 | 1.26 | 95 |
| | 32.40 | 10.99 | | 56.61 | | | | | |
| Example 17 | Powder 8 | Powder 16 | | Powder 23 | 0.47 | 11.5 | 45 | 1.26 | 95 |
| | 43.56 | 15.68 | | 40.76 | | | | | |

The powder 8 had a BET specific surface area higher than other powders, and it could be confirmed that increasing the mixing ratio thereof could increase the BET specific surface area of the powder compositional substance to be obtained.

[Table 21]

| | Composition | | | | | | Green body | | Calcined body density [g/cm³] |
|---|---|---|---|---|---|---|---|---|---|
| | $Y_2O_3$ [mol%] | $Er_2O_3$ [wt%] | $Fe_2O_3$ [wt%] | $Co_3O_4$ [wt%] | $Al_2O_3$ [wt%] | $TiO_2$ [wt%] | Density [g/cm³] | Vickers hardness [HV] | |
| Example 15 | 4.969 | 0.101 (0.034mol%) | 0.039 | 0.002 | 0.04 | 0.011 | 3.30 | 10.9 | 3.27 |
| Example 16 | 4.958 | 0 | 0.081 | 0.004 | 0.03 | 0.022 | 3.28 | 11.1 | 3.28 |

(continued)

| | Composition | | | | | | Green body | | Calcined body density [g/cm³] |
| | $Y_2O_3$ [mol%] | $Er_2O_3$ [wt%] | $Fe_2O_3$ [wt%] | $Co_3O_4$ [wt%] | $Al_2O_3$ [wt%] | $TiO_2$ [wt%] | Density [g/cm³] | Vickers hardness [HV] | |
| Example 17 | 4.912 | 0 | 0.109 | 0.006 | 0.03 | 0.031 | 3.28 | 11.1 | 3.29 |

Measurement example 4

Measurement of the Vickers hardness of the calcined bodies and preparation and evaluation of the sintered bodies were performed as in Measurement example 1. The measurement results of the Vickers hardness are indicated in Table 22, and the evaluation results of the sintered bodies are indicated in Table 23.

[Table 22]

| | | Vickers hardness [HV] | | Hardness difference [HV] |
| | | Average particle size value | Standard deviation | |
| C1calcined body | Example 15 | 50.1 | 2.4 | 3.8 |
| C3calcined body | Example 16 | 51.3 | 3.3 | 5.0 |
| C4calcined body | Example 17 | 51.8 | 3.6 | 5.5 |

It can be confirmed that, compared with Examples 10 to 12, the calcined bodies obtained in Examples 15 to 17 have high Vickers hardness and small standard deviations.

[Table 23]

| | | Color tone | | | Three-point flexural strength (MPa) | Transmittance ratio |
| | | L* | a | b* | | |
| C1 sintered body | Example 15 | 83.9 | -1.8 | 12.0 | 821 | 1.00 |
| C3 sintered body | Example 16 | 78.0 | -0.7 | 18.8 | 851 | 0.88 |
| C4 sintered body | Example 17 | 74.1 | 2.4 | 19.7 | 849 | 0.76 |

Comparison between Examples 10 to 12 and Examples 15 to 17 could confirm that sintered bodies of various color tones could be obtained irrespective of the BET specific surface areas of the powder compositional substances.

Example 18

Powders in mass percentages indicated in the table below were charged in a 200 mL polypropylene container and dry-mixed by stirring the container so as to obtain a powder compositional substance of examples and comparative examples.
The obtained powder compositional substance was formed and calcined as in Example 1 to obtain calcined a calcined body of Example 18. The results of the powder compositional substance are indicated in Table 24, and the results of the green body and the calcined body are indicated in Table 25. Note that in the composition in Table 25, the contents (in terms of oxide) of elements other than zirconia are shown.

[Table 24]

| | Powder compositional substance [wt%] | | | Average crystal grain size [μm] | BET specific surface area [m²/g] | Average granule size [μm] | Bulk density [g/cm³] | T+C phase ratio [%] |
|---|---|---|---|---|---|---|---|---|
| Example 18 | Powder 7 | Powder 16 | Powder 23 | 0.44 | 10.5 | 44 | 1.27 | 95 |
| | 43.56 | 15.68 | 40.76 | | | | | |
| Example 12 | Powder 4 | Powder 16 | Powder 23 | 0.43 | 10.3 | 44 | 1.28 | 95 |
| | 43.61 | 15.68 | 40.71 | | | | | |

In Example 18, the high-intensity percentage was 0.07%, the coarse intensity percentage was 0%, the maximum intensity ratio was 0.148, the minimum intensity ratio was 0.003, and the intensity ratio range was 0.145.

[Table 25]

| | Composition | | | | | Green body | | Calcined body density [g/cm³] |
|---|---|---|---|---|---|---|---|---|
| | $Y_2O_3$ [mol%] | $Fe_2O_3$ [wt%] | $Co_3O_4$ [wt%] | $Al_2O_3$ [wt%] | $TiO_2$ [wt%] | Density [g/cm³] | Vickers hardness [HV] | |
| Example 18 | 4.912 | 0.109 | 0.006 | 0.03 | 0.031 | 3.30 | 11.4 | 3.28 |
| Example 12 | 4.913 | 0.109 | 0.006 | 0.05 | 0.031 | 3.30 | 11.2 | 3.28 |

Measurement example 5

Measurement of the Vickers hardness of the calcined bodies and preparation and evaluation of the sintered bodies were performed as in Measurement example 1. The measurement results of the Vickers hardness are indicated in Table 26, and the evaluation results of the sintered bodies are indicated in Table 27.

[Table 26]

| | | Vickers hardness [HV] | | Hardness difference [HV] |
|---|---|---|---|---|
| | | Average particle size value | Standard deviation | |
| C4 calcined body | Example 18 | 50.3 | 3.1 | 4.0 |
| | Example 12 | 50.7 | 3.3 | 4.4 |

[Table 27]

| | | Color tone | | | Three-point flexural strength (MPa) | Transmittance ratio |
|---|---|---|---|---|---|---|
| | | L* | a | b* | | |
| C4 sintered body | Example 18 | 73.1 | 2.6 | 19.9 | 837 | 0.76 |
| | Example 12 | 73.8 | 3.0 | 19.8 | 839 | 0.76 |

In Examples 12 and 18 in which the alumina content was 0.05 mass% or less, the calcined bodies had similar Vickers hardness irrespective of the alumina content. Moreover, the obtained sintered bodies had similar color tone, three-point flexural strength and transmittance.

Examples 19 to 24 and Comparative Examples 4 to 6

Powders in mass percentages indicated in the table below were charged in a 200 mL polypropylene container and dry-mixed by stirring the container so as to obtain powder compositional substances of examples and comparative examples.

The obtained powder compositional substances were formed and calcined as in Example 1 to obtain calcined bodies of the examples and the comparative examples. The results of the powder compositional substances are indicated in Table 28, and the results of the green bodies and the calcined bodies are indicated in Table 29. Note that in the composition in Table 29, the contents (in terms of oxide) of elements other than zirconia are shown.

[Table 28]

| | Powder compositional substance [wt%] | | | Average crystal grain size [$\mu$m] | BET specific surface area [$m^2$/g] | Average granule size [$\mu$m] | Bulk density [g/$cm^3$] | T+C phase ratio [%] |
|---|---|---|---|---|---|---|---|---|
| Example 19 | Powder 11 | Powder 15 | Powder 22 | 0.43 | 10.3 | 43 | 1.24 | 81 |
| | 30.00 | 1.00 | 69.00 | | | | | |
| Example 20 | Powder 11 | Powder 15 | Powder 22 | 0.45 | 10.4 | 45 | 1.25 | 88 |
| | 70.00 | 1.00 | 29.00 | | | | | |
| Example 21 | Powder 11 | Powder 15 | Powder 22 | 0.45 | 10.4 | 45 | 1.25 | 92 |
| | 90.00 | 1.00 | 9.00 | | | | | |
| Example 22 | Powder 11 | Powder 13 | Powder 22 | 0.46 | 10.5 | 44 | 1.26 | 81 |
| | 30.00 | 1.00 | 69.00 | | | | | |
| Example 23 | Powder 11 | Powder 13 | Powder 22 | 0.42 | 10.5 | 43 | 1.27 | 88 |
| | 70.00 | 1.00 | 29.00 | | | | | |
| Example 24 | Powder 11 | Powder 13 | Powder 22 | 0.43 | 10.5 | 45 | 1.26 | 92 |
| | 90.00 | 1.00 | 9.00 | | | | | |
| Comparative Example 4 | Powder 12 | Powder 15 | Powder 22 | 0.44 | 10.2 | 44 | 1.25 | 81 |
| | 30.00 | 1.00 | 69.00 | | | | | |
| Comparative Example 5 | Powder 12 | Powder 15 | Powder 22 | 0.44 | 10.3 | 44 | 1.23 | 88 |
| | 70.00 | 1.00 | 29.00 | | | | | |
| Comparative Example 6 | Powder 12 | Powder 15 | Powder 22 | 0.44 | 10.3 | 44 | 1.25 | 92 |
| | 90.00 | 1.00 | 9.00 | | | | | |

The high-intensity percentage was 99.24% in Example 22 and 99.33% in Example 24. Furthermore, the coarse intensity percentage was 5.64 in Example 22 and 6.66 in Example 24.

The maximum intensity ratio was 0.127 in Example 22 and 0.140 in Example 24, and the minimum intensity ratio was 0.036 in Example 22 and 0.032 in Example 24. This confirmed that there is a tendency for the intensity ratio range to widen compared to the case in which the M1 element is iron since the intensity ratio range was 0.091 in Example 22 and 0.108 in Example 24.

[Table 29]

| | | Composition | | | | Green body | | Calcined body density [g/cm³] |
|---|---|---|---|---|---|---|---|---|
| | | $Y_2O_3$ [mol%] | NiO [wt%] | $Co_3O_4$ [wt%] | $Al_2O_3$ [wt%] | Density [g/cm³] | Vickers hardness [HV] | |
| Example 19 | | 4.062 | 0.015 | 0.0005 | 0.05 | 3.32 | 11.0 | 3.28 |
| Example 20 | | 4.063 | 0.035 | 0.0005 | 0.05 | 3.32 | 11.2 | 3.29 |
| Example 21 | | 4.064 | 0.045 | 0.0005 | 0.05 | 3.32 | 11.2 | 3.29 |
| Example 22 | | 4.062 | 0.015 | 0.0005 | 0.05 | 3.32 | 11.1 | 3.28 |
| Example 23 | | 4.063 | 0.035 | 0.0005 | 0.05 | 3.32 | 11.5 | 3.29 |
| Example 24 | | 4.064 | 0.045 | 0.0005 | 0.05 | 3.32 | 11.8 | 3.29 |
| Comparative Example 4 | | 4.062 | 0.015 | 0.0005 | 0.05 | 3.32 | 10.0 | 3.34 |
| Comparative Example 5 | | 4.063 | 0.035 | 0.0005 | 0.05 | 3.32 | 10.2 | 3.37 |
| Comparative Example 6 | | 4.064 | 0.045 | 0.0005 | 0.05 | 3.32 | 10.5 | 3.39 |

Compared with the comparative examples, the green bodies of the examples had Vickers hardness as high as 11.0 HV or more, and it could be confirmed that defects rarely occur in these green bodies.

Measurement example 6

Measurement of the Vickers hardness of the calcined bodies and preparation and evaluation of the sintered bodies were performed as in Measurement example 1. The measurement results of the Vickers hardness are indicated in Table 30, and the evaluation results of the sintered bodies are indicated in Table 31.

[Table 30]

| | | Vickers hardness [HV] | | Hardness difference [HV] |
|---|---|---|---|---|
| | | Average particle size value | Standard deviation | |
| C1 calcined body | Example 19 | 50.5 | 2.1 | 4.2 |
| | Example 22 | 50.3 | 2.0 | 4.0 |
| | Comparative Example 4 | 53.6 | 3.1 | 7.3 |
| C3 calcined body | Example 20 | 51.2 | 2.7 | 4.9 |
| | Example 23 | 51.2 | 2.9 | 4.9 |
| | Comparative Example 5 | 60.4 | 3.9 | 14.1 |
| C4 calcined body | Example 21 | 51.8 | 2.9 | 5.5 |
| | Example 24 | 51.5 | 3.0 | 5.2 |
| | Comparative Example 6 | 63.4 | 5.3 | 17.1 |

Among the calcined bodies from which the sintered bodies having similar color tones were obtained, those calcined bodies of the examples had lower hardness and a smaller hardness difference than those calcined bodies of the comparative examples. In addition, it could be confirmed that the standard deviation was small and the lot-to-lot variation in hardness decreased.

[Table 31]

| | | Color tone | | | Three-point flexural strength (MPa) | Transmittance ratio |
|---|---|---|---|---|---|---|
| | | L* | a | b* | | |
| C1 sintered body | Example 19 | 84.8 | 2.0 | 13.5 | 1093 | 0.86 |
| | Example 22 | 85.1 | 1.8 | 13.2 | 1111 | 0.86 |
| | Comparative Example 4 | 84.5 | 2.1 | 13.7 | 1128 | 0.86 |
| C3 sintered body | Example 20 | 77.1 | 5.7 | 17.3 | 1125 | 0.69 |
| | Example 23 | 77.5 | 5.9 | 17.5 | 1091 | 0.69 |
| | Comparative Example 5 | 77.2 | 5.6 | 17.3 | 1089 | 0.69 |
| C4 sintered body | Example 21 | 74.8 | 5.8 | 17.1 | 1105 | 0.64 |
| | Example 24 | 74.9 | 5.6 | 17.0 | 1119 | 0.64 |
| | Comparative Example 6 | 74.6 | 5.6 | 17.1 | 1121 | 0.64 |

It could be confirmed that sintered bodies having similar color tones were obtained from all of the calcined bodies of the examples and the comparative examples.

Examples 25 to 30 and Comparative Examples 7 to 9

Powders in mass percentages indicated in the table below were charged in a 200 mL polypropylene container and dry-mixed by stirring the container so as to obtain powder compositional substances of examples and comparative examples.

The obtained powder compositional substances were compacted and calcined as in Example 1 to obtain calcined bodies of the examples and the comparative examples. The results of the powder compositional substances are indicated in Table 32, and the results of the green bodies and the calcined bodies are indicated in Table 33. Note that in the composition in Table 33, the contents (in terms of oxide) of elements other than zirconia are shown.

[Table 32]

| | Powder compositional substance [wt%] | | | Average crystal grain size [μm] | BET specific surface area [m²/g] | Average granule size [μm] | Bulk density [g/cm³] | T+C phase ratio [%] |
|---|---|---|---|---|---|---|---|---|
| Example 25 | Powder 11 | Powder 18 | Powder 22 | 0.44 | 10.3 | 44 | 1.25 | 81 |
| | 30.00 | 0.40 | 69.60 | | | | | |
| Example 26 | Powder 11 | Powder 18 | Powder 22 | 0.44 | 10.3 | 44 | 1.24 | 88 |
| | 70.00 | 0.40 | 69.60 | | | | | |
| Example 27 | Powder 11 | Powder 18 | Powder 22 | 0.45 | 10.3 | 44 | 1.24 | 92 |
| | 90.00 | 0.40 | 69.60 | | | | | |
| Example 28 | Powder 11 | Powder 17 | Powder 22 | 0.45 | 10.3 | 43 | 1.25 | 81 |
| | 30.00 | 0.40 | 69.60 | | | | | |
| Example 29 | Powder 11 | Powder 17 | Powder 22 | 0.46 | 10.4 | 44 | 1.24 | 88 |
| | 70.00 | 0.40 | 69.60 | | | | | |

(continued)

| | Powder compositional substance [wt%] | | | Average crystal grain size [μm] | BET specific surface area [m²/g] | Average granule size [μm] | Bulk density [g/cm³] | T+C phase ratio [%] |
|---|---|---|---|---|---|---|---|---|
| Example 30 | Powder 11 | Powder 17 | Powder 22 | 0.47 | 10.3 | 44 | 1.26 | 92 |
| | 90.00 | 0.40 | 69.60 | | | | | |
| Comparative Example 7 | Powder 12 | Powder 18 | Powder 22 | 0.43 | 10.4 | 45 | 1.25 | 81 |
| | 30.00 | 0.40 | 69.60 | | | | | |
| Comparative Example 8 | Powder 12 | Powder 18 | Powder 22 | 0.43 | 10.4 | 44 | 1.24 | 88 |
| | 70.00 | 0.40 | 69.60 | | | | | |
| Comparative Example 9 | Powder 12 | Powder 18 | Powder 22 | 0.42 | 10.4 | 44 | 1.26 | 92 |
| | 90.00 | 0.40 | 69.60 | | | | | |

In Example 27, the high-intensity percentage was 99.42%, the coarse intensity percentage was 4.51%, the maximum intensity ratio was 0.125, the minimum intensity ratio was 0.033, and the intensity ratio range was 0.092.

[Table 33]

| | Composition | | | | Green body | | Calcined body density [g/cm³] |
|---|---|---|---|---|---|---|---|
| | $Y_2O_3$ [mol%] | NiO [wt%] | $Mn_3O_4$ [wt%] | $Al_2O_3$ [wt%] | Density [g/cm³] | Vickers hardness [HV] | |
| Example 25 | 4.062 | 0.015 | 0.0002 | 0.05 | 3.32 | 11.2 | 3.28 |
| Example 26 | 4.063 | 0.035 | 0.0002 | 0.05 | 3.32 | 11.0 | 3.29 |
| Example 27 | 4.064 | 0.045 | 0.0002 | 0.05 | 3.32 | 11.2 | 3.29 |
| Example 28 | 4.062 | 0.015 | 0.0002 | 0.05 | 3.32 | 11.0 | 3.28 |
| Example 29 | 4.063 | 0.035 | 0.0002 | 0.05 | 3.32 | 11.2 | 3.29 |
| Example 30 | 4.064 | 0.045 | 0.0002 | 0.05 | 3.32 | 11.4 | 3.29 |
| Comparative Example 7 | 4.062 | 0.015 | 0.0002 | 0.05 | 3.32 | 10.3 | 3.34 |
| Comparative Example 8 | 4.063 | 0.035 | 0.0002 | 0.05 | 3.32 | 10.5 | 3.38 |
| Comparative Example 9 | 4.064 | 0.045 | 0.0002 | 0.05 | 3.32 | 10.5 | 3.39 |

Compared with the comparative examples, all of the green bodies of the examples had Vickers hardness as high as 11.0 HV or more, and it could be confirmed that defects rarely occur in these green bodies.

Measurement example 7

Measurement of the Vickers hardness of the calcined bodies and preparation and evaluation of the sintered bodies were performed as in Measurement example 1. The measurement results of the Vickers hardness are indicated in Table 34, and the evaluation results of the sintered bodies are indicated in Table 35.

[Table 34]

| | | Vickers hardness [HV] | | Hardness difference [HV] |
|---|---|---|---|---|
| | | Average particle size value | Standard deviation | |
| C1 calcined body | Example 25 | 50.1 | 2.1 | 3.8 |
| | Example 28 | 50.2 | 2.0 | 3.9 |
| | Comparative Example 7 | 53.1 | 3.3 | 6.8 |
| C3 calcined body | Example 26 | 50.9 | 2.9 | 4.6 |
| | Example 29 | 50.5 | 2.7 | 4.2 |
| | Comparative Example 8 | 60.4 | 3.8 | 14.1 |
| C4 calcined body | Example 27 | 51.4 | 3.0 | 5.1 |
| | Example 30 | 51.5 | 2.9 | 5.2 |
| | Comparative Example 9 | 63.9 | 5.5 | 17.6 |

Among the calcined bodies from which the sintered bodies having similar color tones were obtained, those calcined bodies of the examples had lower hardness and a smaller hardness difference than those calcined bodies of the comparative examples. In addition, it could be confirmed that the standard deviation was small and the lot-to-lot variation in hardness decreased.

[Table 35]

| | | Color tone | | | Three-point flexural strength (MPa) | Transmittance ratio |
|---|---|---|---|---|---|---|
| | | L* | a | b* | | |
| C1 sintered body | Example 25 | 75.0 | 0.1 | 5.2 | 1110 | 0.83 |
| | Example 28 | 74.6 | 0.2 | 5.0 | 1078 | 0.86 |
| | Comparative Example 7 | 75.2 | 0.4 | 5.5 | 1098 | 0.86 |
| C3 sintered body | Example 26 | 77.6 | 5.3 | 17.2 | 1099 | 0.69 |
| | Example 29 | 77.5 | 5.7 | 17.3 | 1059 | 0.69 |
| | Comparative Example 8 | 77.4 | 5.6 | 17.0 | 1101 | 0.69 |
| C4 sintered body | Example 27 | 74.8 | 5.9 | 17.0 | 1087 | 0.64 |
| | Example 30 | 74.6 | 5.8 | 17.0 | 1082 | 0.64 |
| | Comparative Example 9 | 74.4 | 5.9 | 17.5 | 1096 | 0.64 |

It could be confirmed that sintered bodies having the same color tones were obtained from all of the calcined bodies of the examples and the comparative examples.

The entire contents of the description, the claims and the abstract of Japanese Patent Application No. 2022-149612 filed September 20, 2022 and Japanese Patent Application No. 2023-038003 filed March 10, 2023 are hereby incorporated by reference as the disclosure of the description of the present disclosure.

## Claims

1.   A zirconia compositional substance comprising:

at least one first transition metal element selected from the group consisting of manganese (Mn), iron (Fe), cobalt (Co), nickel (Ni), copper (Cu), molybdenum (Mo), technetium (Tc), ruthenium (Ru), rhodium (Rh), palladium (Pd) and silver (Ag);
a coloring element that is either or both of a lanthanoid rare earth element and a second transition metal element different from the first transition metal element; and

stabilizing element-containing zirconia,

wherein

a content of the first transition metal element is 100 mass ppm or more,
a content of the second transition metal element is less than 100 mass ppm, and
the zirconia compositional substance satisfies either or both of the following conditions:

when a characteristic X-ray of a zirconium element therein and a characteristic X-ray of the first transition metal element are measured, a percentage of measurement points at which a ratio of an intensity of the characteristic X-ray of the first transition metal element to an intensity of the characteristic X-ray of the zirconium element is 0.05 or more is 3% or less of all measurement points; and
when the characteristic X-ray of the zirconium element therein and the characteristic X-ray of the first transition metal element are measured, a distribution width of the ratio of the intensity of the characteristic X-ray of the first transition metal element to the intensity of the characteristic X-ray of the zirconium element is 0.3 or less.

2. The zirconia compositional substance according to Claim 1, wherein, when the characteristic X-ray of the zirconium element therein and the characteristic X-ray of the first transition metal element are measured, the distribution width of the ratio of the intensity of the characteristic X-ray of the first transition metal element to the intensity of the characteristic X-ray of the zirconium element is 0.3 or less.

3. The zirconia compositional substance according to Claim 1 or 2, wherein, when the characteristic X-ray of the zirconium element therein and the characteristic X-ray of the first transition metal element are measured, the percentage of measurement points at which the ratio of the intensity of the characteristic X-ray of the first transition metal element to the intensity of the characteristic X-ray of the zirconium element is 0.05 or more is 3% or less of all measurement points.

4. The zirconia compositional substance according to any one of Claims 1 to 3, wherein the second transition metal element is at least one transition metal element selected from the group consisting of manganese, iron, cobalt, nickel, copper, molybdenum, technetium, ruthenium, rhodium, palladium and silver.

5. The zirconia compositional substance according to any one of Claims 1 to 4, wherein the lanthanoid rare earth element is at least one selected from the group consisting of praseodymium (Pr), neodymium (Nd), samarium (Sm), europium (Eu), gadolinium (Gd), terbium (Tb), erbium (Er) and ytterbium (Yb).

6. The zirconia compositional substance according to any one of Claims 1 to 5, wherein the lanthanoid rare earth element is a lanthanoid rare earth element dissolved in zirconia.

7. The zirconia compositional substance according to any one of Claims 1 to 6, wherein a content of the lanthanoid rare earth element is 1.0 mass% or less.

8. The zirconia compositional substance according to any one of Claims 1 to 7, comprising at least one third transition metal element selected from the group consisting of titanium (Ti), vanadium (V) and niobium (Nb).

9. The zirconia compositional substance according to any one of Claims 1 to 8, comprising at least one selected from the group consisting of alumina ($Al_2O_3$), silica ($SiO_2$) and germania ($Ge_2O_3$).

10. The zirconia compositional substance according to any one of Claims 1 to 9, wherein a BET specific surface area is 8 $m^2/g$ or more and 15 $m^2/g$ or less.

11. The zirconia compositional substance according to any one of Claims 1 to 10, wherein the zirconia compositional substance is a powder.

12. The zirconia compositional substance according to Claim 11, wherein, when $3.0 \pm 0.1$ g of the zirconia compositional substance is packed in a mold having a diameter of 25 mm, uniaxially press-formed at a pressure of 49 MPa and then subjected to a CIP process at a pressure of 196 MPa, a measured density is 3.2 $g/cm^3$ or more.

13. The zirconia compositional substance according to any one of Claims 1 to 10, wherein the zirconia compositional substance is a green body.

14. The zirconia compositional substance according to Claim 13, wherein the zirconia compositional substance has Vickers hardness of 11.0 or more.

15. A method for producing a calcined body, the method comprising a step of calcining the zirconia compositional substance according to any one of Claims 1 to 14.

16. A method for producing a sintered body, the method comprising a step of sintering a calcined body obtained by the production method according to Claim 15.

## INTERNATIONAL SEARCH REPORT

| International application No. |
|---|
| **PCT/JP2023/033785** |

**A. CLASSIFICATION OF SUBJECT MATTER**

*C04B 35/488*(2006.01)i; *A61C 13/083*(2006.01)i; *C01G 25/02*(2006.01)i
FI: C04B35/488; A61C13/083; C01G25/02

According to International Patent Classification (IPC) or to both national classification and IPC

**B. FIELDS SEARCHED**

Minimum documentation searched (classification system followed by classification symbols)

C04B35/488; A61C13/083; C01G25/02

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | WO 2014/104236 A1 (TOSOH CORPORATION) 03 July 2014 (2014-07-03) paragraphs [0081]-[0104] | 1-7, 9-16 |
| A | | 8 |
| A | WO 2022/158491 A1 (TOSOH CORPORATION) 28 July 2022 (2022-07-28) entire text | 1-16 |
| A | JP 2016-60687 A (KURARAY NORITAKE DENTAL INC) 25 April 2016 (2016-04-25) entire text | 1-16 |
| P, A | WO 2023/145766 A1 (TOSOH CORPORATION) 03 August 2023 (2023-08-03) entire text, all drawings | 1-16 |

☐ Further documents are listed in the continuation of Box C. ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **07 November 2023** | **21 November 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2023/033785**

| Patent document cited in search report | | | Publication date (day/month/year) | Patent family member(s) | | | Publication date (day/month/year) |
|---|---|---|---|---|---|---|---|
| WO | 2014/104236 | A1 | 03 July 2014 | US | 2015/0315086 | A1 | |
| | | | | paragraphs [0121]-[0157] | | | |
| | | | | EP | 2939993 | A1 | |
| | | | | CN | 104884408 | A | |
| | | | | KR | 10-2015-0099708 | A | |
| WO | 2022/158491 | A1 | 28 July 2022 | JP | 2022-113137 | A | |
| JP | 2016-60687 | A | 25 April 2016 | (Family: none) | | | |
| WO | 2023/145766 | A1 | 03 August 2023 | JP | 2023-109721 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3892254 A
- US 9428422 B
- JP 2022149612 A
- JP 2023038003 A